# EUROPEAN PATENT APPLICATION

(11) **EP 3 838 912 A1**
(43) Date of publication of application: **23.06.2021**
(21) Application number: 19218611.2
(22) Date of filing: 20.12.2019
(51) Int. Cl.: C07K 5/02, A61P 25/16, C07K 7/64

(54) **RETRO-INVERSO PEPTIDES**

(71) Applicant: Herantis Pharma Oyj, 02600 Espoo (FI)
(72) Inventor: HUTTUNEN, Henri, 00670 Helsinki (FI); BHATTACHARJEE, Arnab, 02760 Espoo (FI); KULESSKAYA, Natalia, 00790 Helsinki (FI)
(74) Representative: Boco IP Oy Ab

(57) **Abstract**

The present disclosure relates to the field of unconventional neurotrophic factors and to the field of treating degenerative, chronic or progressive diseases and disorders, and monogenic hereditary diseases having ER stress as a pathogenic compound. More particularly the disclosure relates to modified peptides, particularly retro-inverso peptides. The disclosure also relates to pharmaceutical compositions comprising said peptides. Further, the disclosure also relates to said peptides, and pharmaceutical compositions for use as a medicament and in the treatment of degenerative, chronic or progressive diseases and disorders, and monogenic hereditary diseases having ER stress as a pathogenic compound as well as to methods for treating said diseases and disorders.

SEQUENCE LISTING

<110> Herantis Pharma Oyj

<120> RETRO-INVERSO PEPTIDES

<130> P4702EP00

<160> 62

<170> BiSSAP 1.3.6

<210> 1
<211> 23
<212> PRT
<213> Artificial Sequence

<220>
<223> Native sequence (L-aa)

<220>
<221> DISULFID
<222> join(17..20)

<400> 1
Met Arg Val Ala Glu Leu Lys Gln Ile Leu His Ser Trp Gly Glu Glu
1               5                   10                  15
Cys Arg Ala Cys Ala Glu Lys
            20

<210> 2
<211> 23
<212> PRT
<213> Artificial Sequence

<220>
<223> Reversed sequence (D-aa)

<220>
<221> DISULFID
<222> join(4..7)

<400> 2
Lys Glu Ala Cys Ala Arg Cys Glu Glu Gly Trp Ser His Leu Ile Gln
1               5                   10                  15
Lys Leu Glu Ala Val Arg Met
            20

<210> 3
<211> 23
<212> PRT
<213> Artificial Sequence

<220>
<223> Native sequence (L-aa)

<220>
<221> DISULFID
<222> join(17..20)

<400> 3
Leu Arg Val Lys Glu Leu Lys Lys Ile Leu Asp Asp Trp Gly Glu Thr
1               5                   10                  15
Cys Lys Gly Cys Ala Glu Lys
            20

<210> 4
<211> 23
<212> PRT
<213> Artificial Sequence

<220>
<223> Reversed sequence (D-aa)

<220>
<221> DISULFID
<222> join(4..7)

<400> 4
Lys Glu Ala Cys Gly Lys Cys Thr Glu Gly Trp Asp Asp Leu Ile Lys
1               5                   10                  15
Lys Leu Glu Lys Val Arg Leu
            20

<210> 5
<211> 11
<212> PRT
<213> Artificial Sequence

<220>
<223> Native sequence (L-aa)

<220>
<221> DISULFID
<222> join(4..7)

<400> 5
Gly Glu Glu Cys Arg Ala Cys Ala Glu Lys Thr
1               5                   10

<210> 6
<211> 11
<212> PRT
<213> Artificial Sequence

<220>
<223> Reversed sequence (D-aa)

<220>
<221> DISULFID
<222> join(5..8)

<400> 6
Thr Lys Glu Ala Cys Ala Arg Cys Glu Glu Gly
1               5                   10

<210> 7
<211> 11
<212> PRT
<213> Artificial Sequence

<220>
<223> Native sequence (L-aa)

<220>
<221> DISULFID
<222> join(4..7)

<400> 7
Gly Glu Thr Cys Lys Gly Cys Ala Glu Lys Ser
1               5                   10

<210> 8
<211> 11
<212> PRT
<213> Artificial Sequence


<220>
<223> Reversed sequence (D-aa)

<220>
<221> DISULFID
<222> join(5..8)

<400> 8
Ser Lys Glu Ala Cys Gly Lys Cys Thr Glu Gly
1               5                   10

<210> 9
<211> 12
<212> PRT
<213> Artificial Sequence


<220>
<223> Native sequence (L-aa)

<220>
<221> DISULFID
<222> join(4..8)

<400> 9
Gly Glu Glu Cys Arg Gly Ala Cys Ala Glu Lys Thr
1               5                   10

<210> 10
<211> 12
<212> PRT
<213> Artificial Sequence


<220>
<223> Reversed sequence (D-aa)

<220>
<221> DISULFID
<222> join(5..9)

<400> 10
Thr Lys Glu Ala Cys Ala Gly Arg Cys Glu Glu Gly
1               5                   10

<210> 11
<211> 12
<212> PRT
<213> Artificial Sequence

<220>
<223> Native sequence (L-aa)

<220>
<221> DISULFID
<222> join(4..8)

<400> 11
Gly Glu Thr Cys Lys Gly Gly Cys Ala Glu Lys Ser
1               5                   10

<210> 12
<211> 12
<212> PRT
<213> Artificial Sequence

<220>
<223> Reversed sequence (D-aa)

<220>
<221> DISULFID
<222> join(5..9)

<400> 12
Ser Lys Glu Ala Cys Gly Gly Lys Cys Thr Glu Gly
1               5                   10

<210> 13
<211> 8
<212> PRT
<213> Artificial Sequence

<220>
<223> Native sequence (L-aa)

<220>
<221> DISULFID
<222> join(3..6)

<400> 13
Glu Glu Cys Arg Ala Cys Ala Glu
1               5

<210> 14
<211> 8
<212> PRT
<213> Artificial Sequence

<220>
<223> Reversed sequence (D-aa)

<220>
<221> DISULFID

<222> join(3..6)

<400> 14
Glu Ala Cys Ala Arg Cys Glu Glu
1               5

<210> 15
<211> 8
<212> PRT
<213> Artificial Sequence

<220>
<223> Native sequence (L-aa)

<220>
<221> DISULFID
<222> join(3..6)

<400> 15
Glu Thr Cys Lys Gly Cys Ala Glu
1               5

<210> 16
<211> 8
<212> PRT
<213> Artificial Sequence

<220>
<223> Reversed sequence (D-aa)

<220>
<221> DISULFID
<222> join(3..6)

<400> 16
Glu Ala Cys Gly Lys Cys Thr Glu
1               5

<210> 17
<211> 5
<212> PRT
<213> Artificial Sequence

<220>
<223> peptide

<220>
<221> VARIANT
<222> 2
<223> X1 = R, K, I, G, A or S

<220>
<221> VARIANT
<222> 3
<223> X2 = absent, G, A, R, K, I or S

<220>
<221> DISULFID
<222> 4
<223> X3 = A, G or S

<400> 17
Cys Xaa Xaa Xaa Cys
1               5

<210> 18
<211> 9
<212> PRT
<213> Artificial Sequence

<220>
<223> peptide

<220>
<221> VARIANT
<222> 2
<223> X4 = E, T, V, D, M or G

<220>
<221> VARIANT
<222> 4
<223> X1 = R, K, I, G, A or S

<220>
<221> VARIANT
<222> 5
<223> X2 = absent, G, A, R, K, I, or S

<220>
<221> VARIANT
<222> 6
<223> X3 = A, G or S

<400> 18
Glu Xaa Cys Xaa Xaa Xaa Cys Ala Glu
1               5

<210> 19
<211> 16
<212> PRT
<213> Artificial Sequence

<220>
<223> peptide

<220>
<221> VARIANT
<222> 1
<223> X5 = absent, H, D, Q, R, Y, N or S

<220>
<221> VARIANT
<222> 2
<223> X6 = absent, S, D, G, N or R

<220>
<221> VARIANT
<222> 3
<223> X7 = absent or W

<220>

<221> VARIANT
<222> 4
<223> X8 = absent or G

<220>
<221> VARIANT
<222> 6
<223> X4 = E, T, V, D, M or G

<220>
<221> VARIANT
<222> 8
<223> X1 = R, K, I, G, A or S

<220>
<221> VARIANT
<222> 9
<223> X2 = absent, G, A R, K, I or S

<220>
<221> VARIANT
<222> 10
<223> X3 = A, G or S

<220>
<221> VARIANT
<222> 14
<223> X9 = absent or K

<220>
<221> VARIANT
<222> 15
<223> X10 = absent, T, S, A, I or N

<220>
<221> VARIANT
<222> 16
<223> X11 = absent, D or E

<400> 19
Xaa Xaa Xaa Xaa Glu Xaa Cys Xaa Xaa Xaa Cys Ala Glu Xaa Xaa Xaa
1               5                   10                  15

<210> 20
<211> 36
<212> PRT
<213> Artificial Sequence

<220>
<223> peptide

<220>
<221> VARIANT
<222> 1
<223> X12 = absent, L, I or V

<220>
<221> VARIANT
<222> 2
<223> X13 = absent or D

<220>
<221> VARIANT
<222> 3
<223> X14 = absent, L or W

<220>
<221> VARIANT
<222> 4
<223> X15 = absent, A, S, T, E or N

<220>
<221> VARIANT
<222> 5
<223> X16 = absent, S or T

<220>
<221> VARIANT
<222> 6
<223> X17 = absent, V or D

<220>
<221> VARIANT
<222> 7
<223> X18 = absent, D or A

<220>
<221> VARIANT
<222> 8
<223> X19 = absent or L

<220>
<221> VARIANT
<222> 9
<223> X20 = absent, R, K, S or W

<220>
<221> VARIANT
<222> 10
<223> X21 = absent or K

<220>
<221> VARIANT
<222> 11
<223> X22 = absent, M, L, I or V

<220>
<221> VARIANT
<222> 12
<223> X23 = absent or R

<220>
<221> VARIANT
<222> 13
<223> X24 = A, K, T, L or V

<220>
<221> VARIANT
<222> 18
<223> X25 = Q, K or R

<220>
<221> VARIANT

<222> 19
<223> X26 = I or V

<220>
<221> VARIANT
<222> 21
<223> X5 = H, D, Q, R, Y, N or S

<220>
<221> VARIANT
<222> 22
<223> X6 = absent, S, D, G, N or R

<220>
<221> VARIANT
<222> 23
<223> X7 = absent or W

<220>
<221> VARIANT
<222> 24
<223> X8 = absent or G

<220>
<221> VARIANT
<222> 26
<223> X4 = E, T, V, D, M or G

<220>
<221> VARIANT
<222> 28
<223> X1 = R, K, I, G, A or S

<220>
<221> VARIANT
<222> 29
<223> X2 = absent, G, A, R, K, I or S

<220>
<221> VARIANT
<222> 30
<223> X3 = A, G or S

<220>
<221> VARIANT
<222> 34
<223> X9 = absent or K

<220>
<221> VARIANT
<222> 35
<223> X10 = absent, or T, S, A, I or N

<220>
<221> VARIANT
<222> 36
<223> X11 = absent, D or E

<400> 20
Xaa Xaa Xaa Xaa Xaa Xaa Xaa Xaa Xaa Xaa Xaa Xaa Val Xaa Glu Leu
1               5                   10                  15
Lys Xaa Ile Leu Xaa Xaa Xaa Xaa Glu Xaa Cys Xaa Xaa Xaa Cys Ala
            20                  25                  30

Glu Xaa Xaa Xaa
        35

<210> 21
<211> 37
<212> PRT
<213> Homo sapiens

<220>
<223> native human CDNF peptide

<400> 21
Arg Val Ala Glu Leu Lys Gln Ile Leu His Ser Trp Gly Glu Glu Cys
1               5                   10                  15
Arg Ala Cys Ala Glu Lys Thr Asp Tyr Val Asn Leu Ile Gln Glu Leu
            20                  25                  30
Ala Pro Lys Tyr Ala
        35

<210> 22
<211> 37
<212> PRT
<213> Homo sapiens

<220>
<223> native human MANF peptide

<400> 22
Arg Val Lys Glu Leu Lys Lys Ile Leu Asp Asp Trp Gly Glu Thr Cys
1               5                   10                  15
Lys Gly Cys Ala Glu Lys Ser Asp Tyr Ile Arg Lys Ile Asn Glu Leu
            20                  25                  30
Met Pro Lys Tyr Ala
        35

<210> 23
<211> 32
<212> PRT
<213> Artificial Sequence

<220>
<223> peptide

<220>
<221> VARIANT
<222> 1
<223> X16 = absent, S or T

<220>
<221> VARIANT
<222> 2
<223> X17 = absent, V or D

<220>
<221> VARIANT
<222> 3
<223> X18 = absent, D or A

<220>
<221> VARIANT

<222> 4
<223> X19 = absent or L

<220>
<221> VARIANT
<222> 5
<223> X20 = absent, R, K, S or W

<220>
<221> VARIANT
<222> 6
<223> X21 = absent or K

<220>
<221> VARIANT
<222> 7
<223> X22 = absent, M, L, I or V

<220>
<221> VARIANT
<222> 8
<223> X23 = absent or R

<220>
<221> VARIANT
<222> 10
<223> X24 = A, K, T, L or V

<220>
<221> VARIANT
<222> 14
<223> X25 = Q, K or R

<220>
<221> VARIANT
<222> 15
<223> X26 = I or V

<220>
<221> VARIANT
<222> 17
<223> X5 = absent, H, D, Q, R, Y, N or S

<220>
<221> VARIANT
<222> 18
<223> X6 = absent, S, D, G, N or R

<220>
<221> VARIANT
<222> 19
<223> X7 = absent or W

<220>
<221> VARIANT
<222> 20
<223> X8 = absent or G

<220>
<221> VARIANT
<222> 22
<223> X4 = E, T, V, D, M or G

<220>
<221> VARIANT
<222> 24
<223> X1 = R, K, I, G, A or S

<220>
<221> VARIANT
<222> 25
<223> X2 = absent, G, A, R, K, I or S

<220>
<221> VARIANT
<222> 26
<223> X3 = A, G or S

<220>
<221> VARIANT
<222> 30
<223> X9 = absent or K

<220>
<221> VARIANT
<222> 31
<223> X10 = absent, T, S, A, I or N

<220>
<221> VARIANT
<222> 32
<223> X11 = absent, D or E

<400> 23
Xaa Xaa Xaa Xaa Xaa Xaa Xaa Xaa Val Xaa Glu Leu Lys Xaa Xaa Leu
1               5                   10                  15
Xaa Xaa Xaa Xaa Glu Xaa Cys Xaa Xaa Xaa Cys Ala Glu Xaa Xaa Xaa
            20                  25                  30

<210> 24
<211> 32
<212> PRT
<213> Artificial Sequence

<220>
<223> peptide

<220>
<221> VARIANT
<222> 1
<223> X15 = absent, A, S, T, E or N

<220>
<221> VARIANT
<222> 2
<223> X16 = absent, S or T

<220>
<221> VARIANT
<222> 3
<223> X17 = absent, V or D

<220>

<221> VARIANT
<222> 4
<223> X18 = absent, D or A

<220>
<221> VARIANT
<222> 5
<223> X19 = absent or L

<220>
<221> VARIANT
<222> 6
<223> X20 = absent, R, K, S or W

<220>
<221> VARIANT
<222> 7
<223> X21 = absent or K

<220>
<221> VARIANT
<222> 8
<223> X22 = absent, M, L, I or V

<220>
<221> VARIANT
<222> 9
<223> X23 = absent or R

<220>
<221> VARIANT
<222> 9
<223> X23 = absent or R

<220>
<221> VARIANT
<222> 11
<223> X24 = A, K, T, L or V

<220>
<221> VARIANT
<222> 15
<223> X25 = Q, K or R

<220>
<221> VARIANT
<222> 16
<223> X26 = I or V

<220>
<221> VARIANT
<222> 18
<223> X5 = absent, H, D, Q, R, Y, N or S

<220>
<221> VARIANT
<222> 19
<223> X6 = absent, S, D, G, N or R

<220>
<221> VARIANT
<222> 20
<223> X7 = absent or W

<220>
<221> VARIANT
<222> 21
<223> X8 = absent or G

<220>
<221> VARIANT
<222> 23
<223> X4 = E, T, V, D, M or G

<220>
<221> VARIANT
<222> 25
<223> X1 = R, K, I, G, A or S

<220>
<221> VARIANT
<222> 26
<223> X2 = absent, G, A, R, K, I or S

<220>
<221> VARIANT
<222> 27
<223> X3 = A, G or S

<220>
<221> VARIANT
<222> 31
<223> X9 = absent or K

<400> 24
Xaa Xaa Xaa Xaa Xaa Xaa Xaa Xaa Xaa Val Xaa Glu Leu Lys Xaa Xaa
1               5                   10                  15
Leu Xaa Xaa Xaa Xaa Glu Xaa Cys Xaa Xaa Xaa Cys Ala Glu Xaa Xaa
            20                  25                  30

<210> 25
<211> 32
<212> PRT
<213> Artificial Sequence

<220>
<223> peptide

<220>
<221> VARIANT
<222> 1
<223> X14 = absent, L or W

<220>
<221> VARIANT
<222> 2
<223> X15 = absent, A, S, T, E or N

<220>
<221> VARIANT
<222> 3
<223> X16 = absent, S or T

<220>
<221> VARIANT
<222> 4
<223> X17 = absent, V or D

<220>
<221> VARIANT
<222> 5
<223> X18 = absent, D or A

<220>
<221> VARIANT
<222> 6
<223> X19 = absent or L

<220>
<221> VARIANT
<222> 7
<223> X20 = absent, R, K, S or W

<220>
<221> VARIANT
<222> 8
<223> X21 = absent or K

<220>
<221> VARIANT
<222> 9
<223> X22 = absent, M, L, I or V

<220>
<221> VARIANT
<222> 10
<223> X23 = absent or R

<220>
<221> VARIANT
<222> 12
<223> X24 = A, K, T, L or V

<220>
<221> VARIANT
<222> 16
<223> X25 = Q, K or R

<220>
<221> VARIANT
<222> 17
<223> X26 = I or V

<220>
<221> VARIANT
<222> 19
<223> X5 = absent, H, D, Q, R, Y, N or S

<220>
<221> VARIANT
<222> 20
<223> X6 = absent, S, D, G, N or R

<220>
<221> VARIANT
<222> 21

<223> X7 = absent or W

<220>
<221> VARIANT
<222> 22
<223> X8 = absent or G

<220>
<221> VARIANT
<222> 24
<223> X4 = E, T, V, D, M or G

<220>
<221> VARIANT
<222> 26
<223> X1 = R, K, I, G, A or S

<220>
<221> VARIANT
<222> 27
<223> X2 = absent, G, A, R, K, I or S

<220>
<221> VARIANT
<222> 28
<223> X3 = A, G or S

<220>
<221> VARIANT
<222> 32
<223> X9 = absent or K

<400> 25
Xaa Xaa Xaa Xaa Xaa Xaa Xaa Xaa Xaa Xaa Val Xaa Glu Leu Lys Xaa
1               5                   10                  15
Xaa Leu Xaa Xaa Xaa Xaa Glu Xaa Cys Xaa Xaa Xaa Cys Ala Glu Xaa
            20                  25                  30

<210> 26
<211> 32
<212> PRT
<213> Artificial Sequence

<220>
<223> peptide

<220>
<221> VARIANT
<222> 1
<223> X13 = absent or D

<220>
<221> VARIANT
<222> 2
<223> X14 = absent, L or W

<220>
<221> VARIANT
<222> 3
<223> X15 = absent, A, S, T, E or N

<220>
<221> VARIANT
<222> 4
<223> X16 = absent, S or T

<220>
<221> VARIANT
<222> 5
<223> X17 = absent, V or D

<220>
<221> VARIANT
<222> 6
<223> X18 = absent, D or A

<220>
<221> VARIANT
<222> 7
<223> X19 = absent or L

<220>
<221> VARIANT
<222> 8
<223> X20 = absent, R, K, S or W

<220>
<221> VARIANT
<222> 9
<223> X21 = absent or K

<220>
<221> VARIANT
<222> 10
<223> X22 = absent, M, L, I or V

<220>
<221> VARIANT
<222> 11
<223> X23 = absent or R

<220>
<221> VARIANT
<222> 13
<223> X24 = A, K, T, L or V

<220>
<221> VARIANT
<222> 17
<223> X25 = Q, K or R

<220>
<221> VARIANT
<222> 18
<223> X26 = I or V

<220>
<221> VARIANT
<222> 20
<223> X5 = absent, H, D, Q, R, Y, N or S

<220>
<221> VARIANT

<222> 21
<223> X6 = absent, S, D, G, N or R

<220>
<221> VARIANT
<222> 22
<223> X7 = absent or W

<220>
<221> VARIANT
<222> 23
<223> X8 = absent or G

<220>
<221> VARIANT
<222> 25
<223> X4 = E, T, V, D, M or G

<220>
<221> VARIANT
<222> 27
<223> X1 = R, K, I, G, A or S

<220>
<221> VARIANT
<222> 28
<223> X2 = absent, G, A, R, K, I or S

<220>
<221> VARIANT
<222> 29
<223> X3 = A, G or S

<400> 26
Xaa Xaa Xaa Xaa Xaa Xaa Xaa Xaa Xaa Xaa Xaa Val Xaa Glu Leu Lys
1               5                   10                  15
Xaa Xaa Leu Xaa Xaa Xaa Xaa Glu Xaa Cys Xaa Xaa Xaa Cys Ala Glu
            20                  25                  30

<210> 27
<211> 4
<212> PRT
<213> Artificial Sequence

<220>
<223> peptide

<400> 27
Lys Asp Glu Leu
1

<210> 28
<211> 4
<212> PRT
<213> Artificial Sequence

<220>
<223> peptide

<400> 28
Cys Arg Ala Cys
1

<210> 29
<211> 4
<212> PRT
<213> Artificial Sequence

<220>
<223> peptide

<400> 29
Cys Lys Gly Cys
1

<210> 30
<211> 4
<212> PRT
<213> Artificial Sequence

<220>
<223> peptide

<220>
<221> VARIANT
<222> 2
<223> X1 = K or R

<220>
<221> VARIANT
<222> 3
<223> X2 = G or A

<400> 30
Cys Xaa Xaa Cys
1

<210> 31
<211> 8
<212> PRT
<213> Artificial Sequence

<220>
<223> peptide

<400> 31
Glu Thr Cys Lys Gly Cys Ala Glu
1               5

<210> 32
<211> 6
<212> PRT
<213> Artificial Sequence

<220>
<223> peptide

<400> 32

Thr Cys Lys Gly Cys Ala
1               5

<210> 33
<211> 187
<212> PRT
<213> Homo sapiens


<220>
<223> full length CDNF (NCBI Reference Sequence: NP_001025125.2)

<400> 33
Met Trp Cys Ala Ser Pro Val Ala Val Val Ala Phe Cys Ala Gly Leu
1               5                   10                  15
Leu Val Ser His Pro Val Leu Thr Gln Gly Gln Glu Ala Gly Gly Arg
            20                  25                  30
Pro Gly Ala Asp Cys Glu Val Cys Lys Glu Phe Leu Asn Arg Phe Tyr
        35                  40                  45
Lys Ser Leu Ile Asp Arg Gly Val Asn Phe Ser Leu Asp Thr Ile Glu
    50                  55                  60
Lys Glu Leu Ile Ser Phe Cys Leu Asp Thr Lys Gly Lys Glu Asn Arg
65                  70                  75                  80
Leu Cys Tyr Tyr Leu Gly Ala Thr Lys Asp Ala Ala Thr Lys Ile Leu
                85                  90                  95
Ser Glu Val Thr Arg Pro Met Ser Val His Met Pro Ala Met Lys Ile
            100                 105                 110
Cys Glu Lys Leu Lys Lys Leu Asp Ser Gln Ile Cys Glu Leu Lys Tyr
        115                 120                 125
Glu Lys Thr Leu Asp Leu Ala Ser Val Asp Leu Arg Lys Met Arg Val
    130                 135                 140
Ala Glu Leu Lys Gln Ile Leu His Ser Trp Gly Glu Glu Cys Arg Ala
145                 150                 155                 160
Cys Ala Glu Lys Thr Asp Tyr Val Asn Leu Ile Gln Glu Leu Ala Pro
                165                 170                 175
Lys Tyr Ala Ala Thr His Pro Lys Thr Glu Leu
            180                 185

<210> 34
<211> 182
<212> PRT
<213> Homo sapiens


<220>
<223> full length MANF (NCBI Reference Sequence: NP_006001.5)

<400> 34
Met Arg Arg Met Trp Ala Thr Gln Gly Leu Ala Val Ala Leu Ala Leu
1               5                   10                  15
Ser Val Leu Pro Gly Ser Arg Ala Leu Arg Pro Gly Asp Cys Glu Val
            20                  25                  30
Cys Ile Ser Tyr Leu Gly Arg Phe Tyr Gln Asp Leu Lys Asp Arg Asp
        35                  40                  45
Val Thr Phe Ser Pro Ala Thr Ile Glu Asn Glu Leu Ile Lys Phe Cys
    50                  55                  60
Arg Glu Ala Arg Gly Lys Glu Asn Arg Leu Cys Tyr Tyr Ile Gly Ala
65                  70                  75                  80
Thr Asp Asp Ala Ala Thr Lys Ile Ile Asn Glu Val Ser Lys Pro Leu
                85                  90                  95
Ala His His Ile Pro Val Glu Lys Ile Cys Glu Lys Leu Lys Lys Lys
            100                 105                 110
Asp Ser Gln Ile Cys Glu Leu Lys Tyr Asp Lys Gln Ile Asp Leu Ser

115                 120                 125
Thr Val Asp Leu Lys Lys Leu Arg Val Lys Glu Leu Lys Lys Ile Leu
    130                 135                 140
Asp Asp Trp Gly Glu Thr Cys Lys Gly Cys Ala Glu Lys Ser Asp Tyr
145                 150                 155                 160
Ile Arg Lys Ile Asn Glu Leu Met Pro Lys Tyr Ala Pro Lys Ala Ala
                165                 170                 175
Ser Ala Arg Thr Asp Leu
            180

<210> 35
<211> 49
<212> PRT
<213> Homo sapiens

<400> 35
Thr Leu Asp Leu Ala Ser Val Asp Leu Arg Lys Met Arg Val Ala Glu
1               5                   10                  15
Leu Lys Gln Ile Leu His Ser Trp Gly Glu Glu Cys Arg Ala Cys Ala
            20                  25                  30
Glu Lys Thr Asp Tyr Val Asn Leu Ile Gln Glu Leu Ala Pro Lys Tyr
        35                  40                  45
Ala

<210> 36
<211> 37
<212> PRT
<213> Homo sapiens

<400> 36
Arg Val Ala Glu Leu Lys Gln Ile Leu His Ser Trp Gly Glu Glu Cys
1               5                   10                  15
Arg Ala Cys Ala Glu Lys Thr Asp Tyr Val Asn Leu Ile Gln Glu Leu
            20                  25                  30
Ala Pro Lys Tyr Ala
        35

<210> 37
<211> 35
<212> PRT
<213> Homo sapiens

<400> 37
Thr Leu Asp Leu Ala Ser Val Asp Leu Arg Lys Met Arg Val Ala Glu
1               5                   10                  15
Leu Lys Gln Ile Leu His Ser Trp Gly Glu Glu Cys Arg Ala Cys Ala
            20                  25                  30
Glu Lys Thr
        35

<210> 38
<211> 32
<212> PRT
<213> Homo sapiens

<400> 38
Leu Ala Ser Val Asp Leu Arg Lys Met Arg Val Ala Glu Leu Lys Gln
1               5                   10                  15

Ile Leu His Ser Trp Gly Glu Glu Cys Arg Ala Cys Ala Glu Lys Thr
20                  25                  30

<210> 39
<211> 49
<212> PRT
<213> Homo sapiens

<400> 39
Gln Ile Asp Leu Ser Thr Val Asp Leu Lys Lys Leu Arg Val Lys Glu
1               5                   10                  15
Leu Lys Lys Ile Leu Asp Asp Trp Gly Glu Thr Cys Lys Gly Cys Ala
            20                  25                  30
Glu Lys Ser Asp Tyr Ile Arg Lys Ile Asn Glu Leu Met Pro Lys Tyr
        35                  40                  45
Ala

<210> 40
<211> 37
<212> PRT
<213> Homo sapiens

<400> 40
Arg Val Lys Glu Leu Lys Lys Ile Leu Asp Asp Trp Gly Glu Thr Cys
1               5                   10                  15
Lys Gly Cys Ala Glu Lys Ser Asp Tyr Ile Arg Lys Ile Asn Glu Leu
            20                  25                  30
Met Pro Lys Tyr Ala
        35

<210> 41
<211> 35
<212> PRT
<213> Homo sapiens

<400> 41
Gln Ile Asp Leu Ser Thr Val Asp Leu Lys Lys Leu Arg Val Lys Glu
1               5                   10                  15
Leu Lys Lys Ile Leu Asp Asp Trp Gly Glu Thr Cys Lys Gly Cys Ala
            20                  25                  30
Glu Lys Ser
        35

<210> 42
<211> 32
<212> PRT
<213> Homo sapiens

<400> 42
Leu Ser Thr Val Asp Leu Lys Lys Leu Arg Val Lys Glu Leu Lys Lys
1               5                   10                  15
Ile Leu Asp Asp Trp Gly Glu Thr Cys Lys Gly Cys Ala Glu Lys Ser
            20                  25                  30

<210> 43
<211> 61
<212> PRT
<213> Homo sapiens

<220>
<223> Human CDNF (NP_001025125.2)

<400> 43
Lys Tyr Glu Lys Thr Leu Asp Leu Ala Ser Val Asp Leu Arg Lys Met
1               5                   10                  15
Arg Val Ala Glu Leu Lys Gln Ile Leu His Ser Trp Gly Glu Glu Cys
            20                  25                  30
Arg Ala Cys Ala Glu Lys Thr Asp Tyr Val Asn Leu Ile Gln Glu Leu
        35                  40                  45
Ala Pro Lys Tyr Ala Ala Thr His Pro Lys Thr Glu Leu
    50                  55                  60

<210> 44
<211> 61
<212> PRT
<213> Equus

<220>
<223> Horse CDNF (XP_001498617.2)

<400> 44
Lys Tyr Glu Lys Lys Leu Asp Leu Ala Ser Val Asp Leu Leu Lys Met
1               5                   10                  15
Arg Val Ala Glu Leu Lys Gln Ile Leu Asn Ser Trp Gly Glu Glu Cys
            20                  25                  30
Arg Ala Cys Ala Glu Lys Ser Asp Tyr Val Asn Leu Ile Lys Glu Leu
        35                  40                  45
Ala Pro Lys Tyr Ala Ala Met His Pro Lys Thr Glu Leu
    50                  55                  60

<210> 45
<211> 61
<212> PRT
<213> Bos

<220>
<223> Bison CDNF (XP_010858254.1)

<400> 45
Lys Tyr Glu Lys Lys Leu Asp Leu Ala Ser Val Asp Leu Ser Lys Met
1               5                   10                  15
Arg Val Ala Glu Leu Lys Gln Ile Leu His Gly Trp Gly Glu Glu Cys
            20                  25                  30
Arg Ala Cys Ala Glu Lys Thr Asp Tyr Val Asn Leu Ile Lys Glu Leu
        35                  40                  45
Ala Pro Lys Tyr Ala Ala Thr His Pro Gln Thr Glu Leu
    50                  55                  60

<210> 46
<211> 61
<212> PRT
<213> Sus

<220>
<223> Pig CDNF (XP_003130787.1)

<400> 46
Lys Tyr Glu Lys Lys Leu Asp Leu Ala Ser Val Asp Leu Ser Lys Met
1               5                   10                  15
Arg Val Ala Glu Leu Lys Gln Ile Leu Tyr Ser Trp Gly Glu Glu Cys
            20                  25                  30
Arg Ala Cys Ala Glu Lys Thr Asp Tyr Val Asn Leu Ile Lys Glu Leu
        35                  40                  45
Ala Pro Lys Tyr Thr Glu Thr Pro Pro Gln Thr Glu Leu
    50                  55                  60

<210> 47
<211> 61
<212> PRT
<213> Canis

<220>
<223> Dog CDNF (XP_848954.2)

<400> 47
Lys Tyr Glu Lys Lys Leu Asp Leu Ala Ser Val Asp Leu Ser Lys Met
1               5                   10                  15
Arg Val Ala Glu Leu Lys Gln Ile Leu His Ser Trp Gly Glu Glu Cys
            20                  25                  30
Ile Ala Cys Ala Glu Lys Thr Asp Tyr Val Asn Leu Ile Thr Glu Leu
        35                  40                  45
Ala Pro Lys Tyr Ala Ala Ala His Pro Lys Thr Glu Leu
    50                  55                  60

<210> 48
<211> 61
<212> PRT
<213> Musculus

<220>
<223> Mouse CDNF (NP_808315.1)

<400> 48
Lys Tyr Gly Lys Lys Leu Asp Leu Ala Ser Val Asp Leu Trp Lys Met
1               5                   10                  15
Arg Val Ala Glu Leu Lys Gln Ile Leu Gln Arg Trp Gly Glu Glu Cys
            20                  25                  30
Arg Ala Cys Ala Glu Lys Ser Asp Tyr Val Asn Leu Ile Arg Glu Leu
        35                  40                  45
Ala Pro Lys Tyr Val Glu Ile Tyr Pro Gln Thr Glu Leu
    50                  55                  60

<210> 49
<211> 61
<212> PRT
<213> Cricetus

<220>
<223> Hamster CDNF (XP_027261009.1)

<400> 49
Asn Tyr Glu Lys Lys Leu Asp Leu Ala Ser Val Asp Leu Trp Lys Met
1               5                   10                  15

Arg Asp Ala Glu Leu Lys Gln Ile Leu His Ser Trp Gly Glu Glu Cys
                20                  25                  30
Arg Ala Cys Ala Glu Lys Asn Asp Tyr Val Asn Leu Ile Lys Glu Leu
            35                  40                  45
Ala Pro Lys Tyr Val Glu Ile His Pro Gln Ile Glu Leu
    50                  55                  60

<210> 50
<211> 61
<212> PRT
<213> Alligator

<220>
<223> Alligator CDNF (XP_019343086.1)

<400> 50
Lys Tyr Glu Arg Lys Leu Asp Leu Thr Ser Val Asp Leu Ser Lys Met
1               5                   10                  15
Arg Val Ala Glu Leu Arg Lys Ile Leu Asp Ser Trp Gly Glu Val Cys
                20                  25                  30
Lys Ala Cys Ile Glu Lys Thr Glu Phe Val Asn Leu Ile Lys Glu Leu
            35                  40                  45
Ala Pro Lys Tyr Ala Pro Pro Asn Ser Arg Ala Asp Leu
    50                  55                  60

<210> 51
<211> 61
<212> PRT
<213> Delphinus

<220>
<223> Dolphin CDNF (XP_026977721.1)

<400> 51
Lys Tyr Glu Lys Lys Leu Asp Leu Ala Ser Val Asp Leu Ser Lys Met
1               5                   10                  15
Arg Val Ala Glu Leu Lys Gln Ile Leu Tyr Ser Trp Gly Glu Glu Cys
                20                  25                  30
Arg Ala Cys Val Glu Lys Thr Asp Tyr Val Asn Leu Ile Lys Glu Leu
            35                  40                  45
Ala Pro Lys Tyr Thr Ala Thr Tyr Pro Lys Thr Glu Leu
    50                  55                  60

<210> 52
<211> 65
<212> PRT
<213> Danio rerio

<220>
<223> Zebrafish CDNF (NP_001116753.1)

<400> 52
Arg Tyr Glu Arg Leu Val Leu Asp Trp Ser Thr Asp Ala Leu Ser Lys
1               5                   10                  15
Met Arg Ala Leu Glu Leu Lys Arg Val Leu Ala Ser Trp Gly Glu Glu
                20                  25                  30
Cys Arg Ala Cys Leu Glu Lys Ser Glu Phe Ile Ala Leu Ile Gln Glu
            35                  40                  45
Val Ala Pro Lys His Ser Ala Ser Glu His Arg Ala His Thr Glu Glu
    50                  55                  60

Phe
65

<210> 53
<211> 63
<212> PRT
<213> Homo sapiens

<220>
<223> Human MANF (NP_006001.5)

<400> 53
Lys Tyr Asp Lys Gln Ile Asp Leu Ser Thr Val Asp Leu Lys Lys Leu
1               5                   10                  15
Arg Val Lys Glu Leu Lys Lys Ile Leu Asp Asp Trp Gly Glu Thr Cys
            20                  25                  30
Lys Gly Cys Ala Glu Lys Ser Asp Tyr Ile Arg Lys Ile Asn Glu Leu
        35                  40                  45
Met Pro Lys Tyr Ala Pro Lys Ala Ala Ser Ala Arg Thr Asp Leu
    50                  55                  60

<210> 54
<211> 63
<212> PRT
<213> Equus

<220>
<223> Horse MANF (NP_001184244.1)

<400> 54
Lys Tyr Asp Lys Gln Ile Asp Leu Ser Thr Val Asp Leu Lys Lys Leu
1               5                   10                  15
Arg Val Lys Glu Leu Lys Lys Ile Leu Asp Asp Trp Gly Glu Thr Cys
            20                  25                  30
Lys Gly Cys Ala Glu Lys Ser Asp Tyr Ile Arg Lys Ile Asn Glu Leu
        35                  40                  45
Met Pro Lys Tyr Ala Pro Lys Ala Ala Ser Ser Arg Thr Asp Leu
    50                  55                  60

<210> 55
<211> 63
<212> PRT
<213> Bison

<220>
<223> Bison MANF (XP_010850093.1)

<400> 55
Lys Tyr Asp Lys Gln Ile Asp Leu Ser Thr Val Asp Leu Lys Lys Leu
1               5                   10                  15
Arg Val Lys Glu Leu Lys Lys Ile Leu Asp Asp Trp Gly Glu Thr Cys
            20                  25                  30
Lys Gly Cys Ala Glu Lys Ser Asp Tyr Ile Arg Lys Ile Asn Glu Leu
        35                  40                  45
Met Pro Lys Tyr Ala Pro Lys Ala Ala Ser Ser Arg Thr Asp Leu
    50                  55                  60

<210> 56
<211> 63
<212> PRT

<213> Sus

<220>
<223> Pig MANF (NP_001231584.1)

<400> 56
Lys Tyr Asp Lys Gln Ile Asp Leu Ser Thr Val Asp Leu Lys Lys Leu
1               5                   10                  15
Arg Val Lys Glu Leu Lys Lys Ile Leu Asp Asp Trp Gly Glu Thr Cys
            20                  25                  30
Lys Gly Cys Ala Glu Lys Ser Asp Tyr Ile Arg Lys Ile Asn Glu Leu
        35                  40                  45
Met Pro Lys Tyr Ala Pro Lys Ala Ala Ser Ser Arg Thr Asp Leu
    50                  55                  60

<210> 57
<211> 63
<212> PRT
<213> Canis

<220>
<223> Dog MANF (XP_003639808.2)

<400> 57
Lys Tyr Asp Lys Gln Ile Asp Leu Arg Thr Val Asp Leu Lys Lys Leu
1               5                   10                  15
Arg Val Arg Glu Leu Lys Lys Ile Leu Asp Asp Trp Gly Glu Thr Cys
            20                  25                  30
Lys Gly Cys Ala Glu Lys Ser Asp Tyr Ile Arg Lys Ile Asn Glu Leu
        35                  40                  45
Met Pro Lys Tyr Ala Pro Lys Ala Ala Ser Ser Arg Thr Asp Leu
    50                  55                  60

<210> 58
<211> 63
<212> PRT
<213> Musculus

<220>
<223> Mouse MANF (NP_083379.2)

<400> 58
Lys Tyr Asp Lys Gln Ile Asp Leu Ser Thr Val Asp Leu Lys Lys Leu
1               5                   10                  15
Arg Val Lys Glu Leu Lys Lys Ile Leu Asp Asp Trp Gly Glu Met Cys
            20                  25                  30
Lys Gly Cys Ala Glu Lys Ser Asp Tyr Ile Arg Lys Ile Asn Glu Leu
        35                  40                  45
Met Pro Lys Tyr Ala Pro Lys Ala Ala Ser Ala Arg Thr Asp Leu
    50                  55                  60

<210> 59
<211> 63
<212> PRT
<213> Cricetus

<220>
<223> Hamster MANF (RLQ67668)

<400> 59
Lys Tyr Asp Lys Gln Ile Asp Leu Ser Thr Val Asp Leu Lys Lys Leu
1               5                   10                  15
Arg Val Lys Glu Leu Lys Lys Ile Leu Asp Asp Trp Gly Glu Met Cys
            20                  25                  30
Lys Gly Cys Ala Glu Lys Ser Asp Tyr Ile Arg Lys Ile Asn Glu Leu
        35                  40                  45
Met Pro Lys Tyr Ala Pro Lys Ala Ala Ser Ala Arg Thr Asp Leu
    50                  55                  60

<210> 60
<211> 63
<212> PRT
<213> Alligator

<220>
<223> Alligator MANF (XP_014455597.1)

<400> 60
Lys Tyr Asp Lys Gln Ile Asp Leu Ser Thr Val Asp Leu Lys Lys Leu
1               5                   10                  15
Arg Val Lys Glu Leu Lys Lys Ile Leu Asp Asp Trp Gly Glu Thr Cys
            20                  25                  30
Lys Gly Cys Ala Glu Lys Ser Asp Tyr Ile Arg Lys Ile Asn Glu Leu
        35                  40                  45
Met Pro Lys Tyr Ala Pro Lys Ala Ala Ser Ser Arg Thr Asp Leu
    50                  55                  60

<210> 61
<211> 63
<212> PRT
<213> Delphinus

<220>
<223> Dolphin MANF (XP_026976745.1)

<400> 61
Lys Tyr Asp Lys Gln Ile Asp Leu Ser Thr Val Asp Leu Lys Lys Leu
1               5                   10                  15
Arg Val Lys Glu Leu Lys Lys Ile Leu Asp Asp Trp Gly Glu Thr Cys
            20                  25                  30
Lys Gly Cys Ala Glu Lys Ser Asp Tyr Ile Arg Lys Ile Asn Glu Leu
        35                  40                  45
Met Pro Lys Tyr Ala Pro Lys Ala Ala Ser Ser Arg Thr Asp Leu
    50                  55                  60

<210> 62
<211> 63
<212> PRT
<213> Danio rerio

<220>
<223> Zebrafish MANF (NP_001070097.1)

<400> 62
Lys Tyr Asp Lys Gln Val Asp Leu Ser Ser Val Asp Leu Lys Lys Leu
1               5                   10                  15
Lys Val Lys Asp Leu Lys Lys Ile Leu Glu Glu Trp Gly Glu Ser Cys
            20                  25                  30
Lys Gly Cys Val Glu Lys Ser Asp Phe Ile Arg Lys Ile Asn Glu Leu

         35                  40                  45
Met Pro Lys Tyr Ala Pro Ser Ala Ala Lys Ala Arg Thr Asp Leu
     50                  55                  60

## Description

### FIELD OF THE DISCLOSURE

The present disclosure relates to the field of unconventional neurotrophic factors and endoplasmic reticulum (ER) located proteins and to the field of treating degenerative, chronic or progressive diseases and disorders. More particularly the disclosure relates to retro-inverso peptides. The disclosure also relates to pharmaceutical compositions comprising said peptides. Further, the disclosure also relates to said peptides and pharmaceutical compositions for use as a medicament and in the treatment of degenerative, chronic or progressive diseases and disorders, and monogenic hereditary diseases having ER stress as a pathogenic compound as well as to methods for treating said diseases and disorders.

### BACKGROUND OF THE DISCLOSURE

Neurotrophic factors (NTF) are a subgroup of growth factors that promote survival and differentiation of neurons and have neuroprotective and neurorestorative properties (Hefti, 1994). NTFs are small proteins that support the growth, survival and differentiation of developing and mature neurons, and protect them from injury and toxins. Cerebral dopamine neurotrophic (CDNF), together with its closest relative mesencephalic astrocyte-derived neurotrophic factor (MANF), form a novel family of unconventional NTF that are both structurally and mechanistically distinct from other growth factors (Lindholm and Saarma, 2010; Huttunen and Saarma, 2019). CDNF and MANF are small monomeric proteins with a molecular weight of approximately 18 kDa, mature proteins 161 and 158 amino acids, respectively, that are expressed in the central nervous system but also in non-neuronal tissues. CDNF and MANF are localized mainly to the lumen of endoplasmic reticulum (ER). They contain an N-terminal signal peptide that directs them to the ER. Both CDNF and MANF also contain a C-terminal KDEL (SEQ ID NO: 27)-like ER-retention signal that is typically absent in growth factors destined for secretion. They interact with ER proteins such as BiP/GRP78, modulate unfolded protein response (UPR) signaling and protect from ER stress-induced cell death. Both CDNF and MANF accumulate in the ER lumen in healthy cells and disruption of the C-terminal ER-retention signal results in their secretion. Detectable levels of CDNF and MANF are found in normal human serum, and MANF also in cerebrospinal fluid (CSF). Based on these characteristics, CDNF and MANF are considered to be general stress-protective proteins rather than highly specific neurotrophic factors (Huttunen and Saarma, 2019). MANF has also been described as a cardiomyokine (Glembotski, 2011).

CDNF and MANF are currently the most efficient proteins for the treatment of degenerating dopamine neurons in the rat 6-OHDA model of Parkinson's disease (Lindholm and Saarma, 2010). Both factors potently prevent the 6-OHDA-induced loss of dopamine neurons of the Parkinson's disease-like motor symptoms when applied before the toxin (Lindholm et al., 2007; Voutilainen et al., 2009). More importantly, post-lesion administration of either factor efficiently restored the normal motor behavior and dopaminergic innervations of the striatum when applied at the stage when the 6-OHDA-induced symptoms of the Parkinson's disease are already far-reaching (Lindholm et al., 2007; Voutilainen et al., 2011). CDNF protects and repairs dopamine neurons also in mouse MPTP model of Parkinson's disease (Airavaara et al., 2012), and in a severe 6-OHDA model it is more efficient than glial cell line-derived neurotrophic factor (GDNF) (Airavaara et al., 2012; Voutilainen et al 2011). The mechanisms behind the neuronal protection for these factors are not fully clear but it has been suggested they activate pathways, which aim at alleviating oxidative- and ER stress and depressing apoptotic cell death. Many pathophysiological conditions including diabetes and neurodegenerative diseases such as Parkinson's disease, Alzheimer's disease (AD) and amyotrophic lateral sclerosis (ALS) are associated with ER stress. Accordingly, the effect of CDNF and MANF has been shown in various central nervous system diseases (WO2009133247; WO2007068803; and Airavaara et al, 2009). Non-cell autonomous mechanisms, including modulation of responses of immune and glial cells, have been shown to contribute to the cell-protective effects of CDNF and MANF (Sousa-Victor et al, 2018). Specifically, CDNF and MANF have been shown to suppress neuroinflammation, which is involved in the pathophysiology of most if not all CNS diseases and injuries (Nadella et al, 2014; Zhao et al, 2013).

CDNF and MANF share ca. 60% amino acid sequence homology, but they have highly similar three-dimensional structures. Both CDNF and MANF are composed of two independently folded domains connected by a flexible loop region (Lindholm and Saarma, 2010). The secondary structure is predominantly α-helical, with five α-helices in the N-terminal domain, and three α-helices in the C-terminal domain. Three disulphide bridges stabilize the N-terminal domain while the C-terminal CRAC (SEQ ID NO: 28) sequence (CKGC (SEQ ID NO: 29) in MANF) forms an internal disulphide bridge. This CXXC (SEQ ID NO: 30) disulphide bridge is found both in CDNF and MANF and plays a central role in the neuroprotective activity of these proteins.

CDNF is expressed in the brain but also in a number of other tissues, including e.g. skeletal muscle, liver, heart, lung, pancreas, testis, salivary gland and enteric nervous system (Lindholm et al, 2007). MANF is also expressed in the brain but also in peripheral tissues such as the pancreas and heart.

Natural peptides such as those disclosed in publications WO 2013/3034805 and WO 2018/202957 can rarely be used as pharmaceutical products.

Document WO 2013/3034805 A1 discloses MANF and CDNF fragments with the length of 4 - 40 amino acids comprising the sequence CKGC (SEQ IN NO: 29) or CRAC (SEQ ID NO: 28). Document WO 2018/202957 A1 discloses CDNF fragments which have the length of at least 50 amino acids. Hellmann et al., 2011, disclose an active C-terminal fragment construct of MANF comprising residues 96-158. Fletcher and Hughes 2006 disclose a CRAC (SEQ ID NO: 28)-containing brain-derived neurotrophic factor (BDNF)-derived peptide with engineered cysteines for loop generation. Therefore, there remains a need in the art for therapeutics with improved metabolic stability and distribution properties.

### BRIEF DESCRIPTION OF THE INVENTION

An aim of the present disclosure is to provide novel modified retro-inverso peptides. Another aim of the present disclosure is to provide uses of said novel peptides.

The present disclosure provides tools with these aforementioned properties by utilizing peptides, especially retro-inverso peptides in a novel and inventive way.

The present inventors found that linear native CDNF and MANF peptides are poor drug molecules due to their quick metabolism and poor distribution when administered to humans or animals, particularly with parenteral administration. Therefore, natural unmodified peptides such as those disclosed in the prior art can rarely be used as pharmaceutical products. The present inventors developed novel stabilized peptides derived from CDNF and MANF that recapitulate the cell-protective effects of CDNF and MANF but are well-suited for non-invasive peripheral administration. The present inventors found that retro-inverso isomerization of CDNF and MANF peptides significantly improves their metabolic stability and distribution properties without loss of their cell-protective activity, as shown by the data of the present disclosure. Also, the present modified peptides are shorter than those disclosed in the prior art.

The biological activity of CDNF/MANF is localized to the C-terminal domain of the protein. The present disclosure describes 8 - 32 amino acid peptides derived from the C-terminal domain of CDNF and MANF, specifically in a retro-inverso isomerized form. Short unmodified octapeptides around the CXXC (SEQ ID NO: 30) motif showed cell-protective activity comparable to full-length CDNF/MANF protein and ability to penetrate cell membranes *in vitro* as disclosed herein.

The present inventors show for the first time ever that retro-inverso isomerization of CDNF/MANF peptides having a CXXC (SEQ ID NO: 30) motif, or a specific type of CXXXC (SEQ ID NO: 17) motif, have significantly improved pharmaceutical properties, e.g. metabolic stability, blood-brain barrier (BBB) penetration and *in vivo* pharmacokinetics. These retro-inverso isomerized peptides may be used for developing medicaments for degenerative, chronic and/or progressive diseases and disorders, or monogenic hereditary diseases having ER stress as a pathogenic component.

The present disclosure provides a peptide having a length of 8 - 32 amino acids or a pharmaceutically acceptable salt thereof comprising a retro-inverso form of an amino acid sequence of C-X₁-X₂-X₃-C (SEQ ID NO: 17),
wherein
X₁ is selected from the group consisting of R, K, I, G, A and S;
X₂ is absent or selected from the group consisting of G, A, R, K, I and S; and
X₃ is selected from the group consisting of A, G and S.

In some aspects, the peptide is a pseudopeptide. In some instances, the peptide has at least one (e.g., 1, 2, 3, 4, 5, 6, or 7) of the following properties: (i) the peptide can dose-dependently protect TH-positive neurons from MPP+ toxicity; (ii) the peptide reduces the number of a-synuclein inclusions in TH-positive neurons; (iii) the peptide has improved stability in plasma compared to its parent counterpart; (iv) the peptide has improved stability in hepatocytes compared to its parent counterpart; or (v) the peptide has improved ability to pass through the blood brain barrier compared to its parent counterpart.

The present disclosure further provides said peptide for use as a medicament.

The present disclosure further provides said peptide for use in the treatment of a degenerative disease or disorder, a chronic disease or disorder, or a progressive disease or disorder, such as a neurodegenerative disease or disorder, or monogenic hereditary diseases having ER stress as a pathogenic component.

The present disclosure further provides a pharmaceutical composition comprising said peptide and at least one of the following: a pharmaceutically acceptable carrier, a pharmaceutically acceptable excipient, preservative, stabilizer and/or diluent.

The present disclosure further provides the pharmaceutical composition for use as a medicament.

The present disclosure further provides a pharmaceutical composition for use in the treatment of a degenerative, chronic, or progressive disease or disorder, such as a neurodegenerative disease or disorder, or monogenic hereditary diseases having ER stress as a pathogenic component.

The present disclosure further provides a method for treating a degenerative, chronic, or progressive disease or disorder, such as a neurodegenerative disease or disorder, or monogenic hereditary diseases having ER stress as a pathogenic component in a subject in need thereof, the method comprising administering to the subject a pharmaceutical composition comprising the aforementioned peptide.

In the following the invention will be described in more detail by means of preferred embodiments.

### BRIEF DESCRIPTION OF THE FIGURES

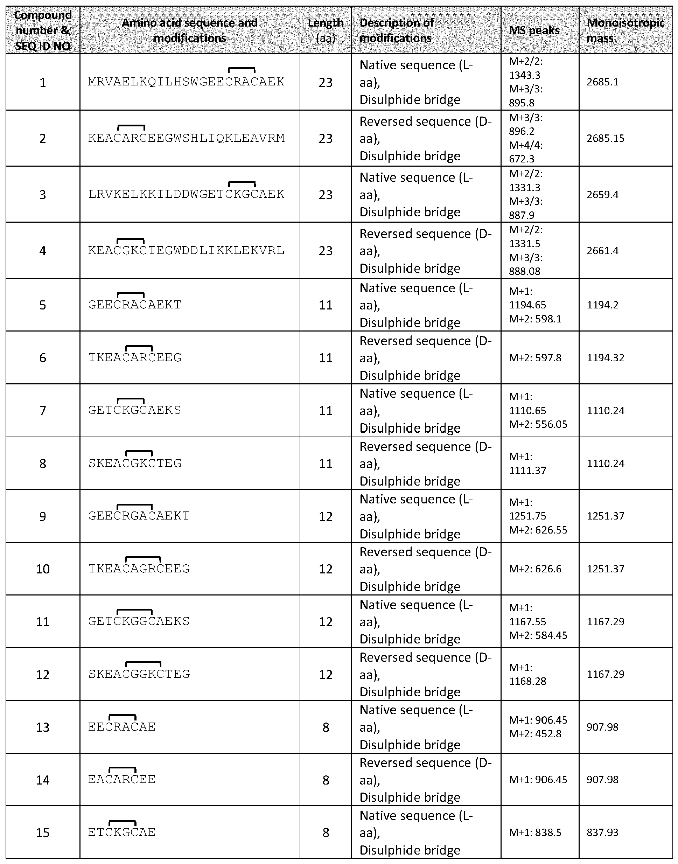

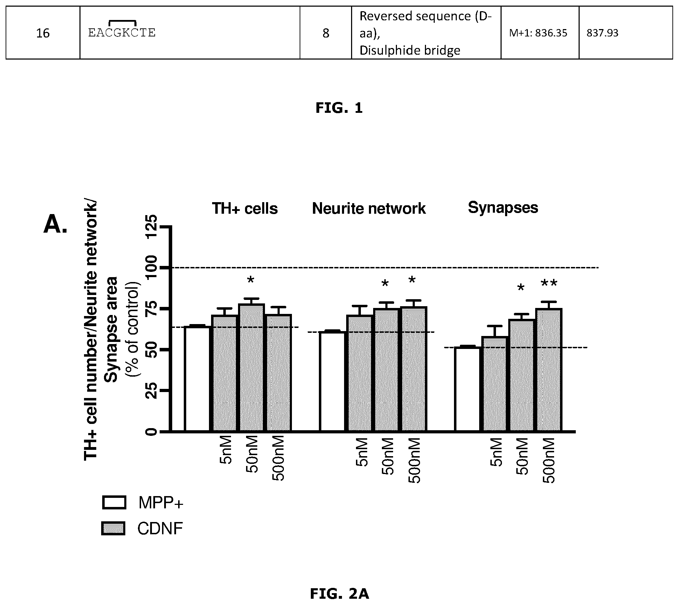

**FIG. 1** shows a list of compounds studied. Compound number, SEQ ID NO, amino acid sequence indicating the Cys-Cys sulphide bond, length of the sequence, description of modification and monoisotronic mass (Da) are presented. Column 5 shows the detailed charged mass peaks seen in the MS spectra and column 6 presents the monoisotropic mass of the compounds.
**FIGs. 2A-2B** show the neuroprotective effects of CDNF on dopaminergic TH (tyrosine hydroxylase)-positive neurons injured with MPP+ (1-methyl-4-phenylpyridinium), and the effect on alpha-synuclein aggregation in TH-positive neurons. Data are expressed as a percentage of control non-injured condition as mean +/- SEM (n=4-6; MPP+ negative control n=122-127 collected across the multiple studies). * p<0.05, ** p<0.01, *** p<0.001, **** p<0.0001 Brown-Forsythe and Welch ANOVA test with post-hoc Dunnett pairwise comparison vs MPP+ negative control.
   **FIG. 2A** shows the number of TH neurons, total neurite network of TH neurons, and the number of synapses on TH neurites in a primary culture of mesencephalic cells after MPP+ injury in the presence of increasing concentrations of rhCDNF. Upper dashed line represents a control level of parameters (100%) obtained from non-injured cells; lower dashed line represents a negative control level of parameters obtained from cells injured with MPP+ without additional treatment with compounds.

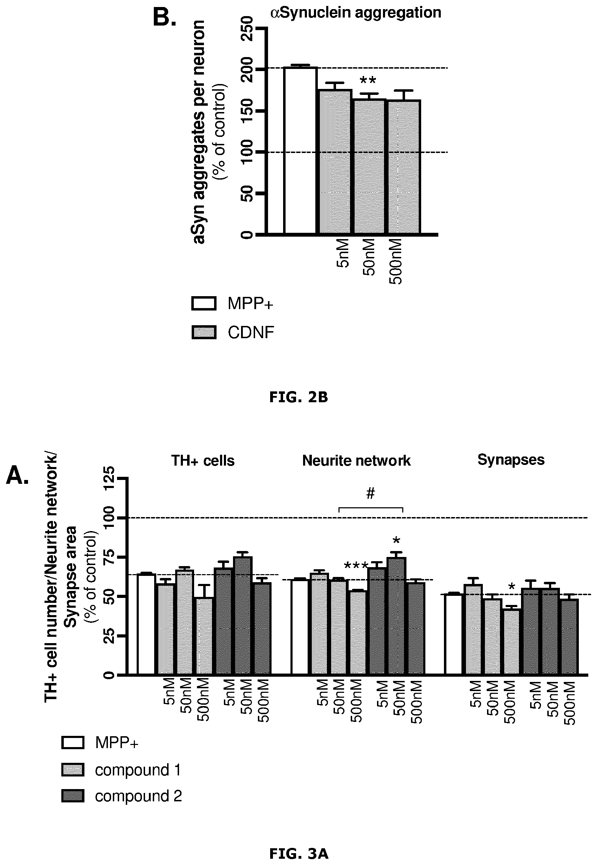

   **FIG. 2B** shows alpha-synuclein (aSyn) aggregation in TH neurons of a primary culture of mesencephalic cells after MPP+ injury in the presence of increasing concentrations of rhCDNF. Upper dashed line represents a negative control level of parameters obtained from cell injured with MPP+ without additional treatment with CDNF; lower dashed line represents a negative control level of parameters (100%) obtained from non-injured cells.
**FIGs. 3A-3H** show the neuroprotective effects of parent and retro-inverso compounds (compounds 1 - 8 having SEQ ID NO:s 1 - 8, respectively) on dopaminergic TH (tyrosine hydroxylase)-positive neurons injured with MPP+ (1-methyl-4-phenylpyridinium), and their effect on alpha-synuclein aggregation in TH-positive neurons. Data are expressed as a percentage of control non-injured condition as mean +/- SEM (n=4-6; MPP+ negative control n=122-127 collected across the multiple studies). * p<0.05, ** p<0.01, *** p<0.001, **** p<0.0001 Brown-Forsythe and Welch ANOVA test with post-hoc Dunnett pairwise comparison vs MPP+ negative control. # p<0.05, ## p<0.01, ### p<0.001, #### p<0.0001 Brown-Forsythe and Welch ANOVA test with post-hoc Dunnett pairwise comparison between parent and corresponding retro-inverso compound in the same concentration.
**FIG. 3A** shows the number of TH neurons, total neurite network of TH neurons, and the number of synapses on TH neurites in a primary culture of mesencephalic cells after MPP+ injury in the presence of compound 1 (SEQ ID NO: 1) and compound 2 (SEQ ID NO: 2). Upper dashed line represents a control level of parameters (100%) obtained from non-injured cells; lower dashed line represents a negative control level of parameters obtained from cells injured with MPP+ without additional treatment with compounds.
   **FIG. 3B** shows alpha-synuclein (aSyn) aggregation in TH neurons of a primary culture of mesencephalic cells after MPP+ injury in the presence of compound 1 (SEQ ID NO: 1) and compound 2 (SEQ ID NO: 2). Upper dashed line represents a negative control level of parameters obtained from cell injured with MPP+ without additional treatment with compounds; lower dashed line represents a negative control level of parameters (100%) obtained from non-injured cells.
   **FIG. 3C** shows the number of TH neurons, total neurite network of TH neurons, and number of synapses on TH neurites in a primary culture of mesencephalic cells after MPP+ injury in the presence of compound 3 (SEQ ID NO: 3) and compound 4 (SEQ ID NO: 4).
   **FIG. 3D** shows alpha-synuclein (aSyn) aggregation in TH neurons of a primary culture of mesencephalic cells after MPP+ injury in the presence of compound 3 (SEQ ID NO: 3) and compound 4 (SEQ ID NO: 4).
   **FIG. 3E** shows the number of TH neurons, total neurite network of TH neurons, and number of synapses on TH neurites in a primary culture of mesencephalic cells after MPP+ injury in the presence of compound 5 (SEQ ID NO: 5) and compound 6 (SEQ ID NO: 6).
   **FIG. 3F** shows alpha-synuclein (aSyn) aggregation in TH neurons of a primary culture of mesencephalic cells after MPP+ injury in the presence of compound 5 (SEQ ID NO: 5) and compound 6 (SEQ ID NO: 6).
   **FIG. 3G** shows the number of TH neurons, total neurite network of TH neurons, and number of synapses on TH neurites in a primary culture of mesencephalic cells after MPP+ injury in the presence of compound 7 (SEQ ID NO: 7) and compound 8 (SEQ ID NO: 8).
   **FIG. 3H** shows alpha-synuclein (aSyn) aggregation in TH neurons of a primary culture of mesencephalic cells after MPP+ injury in the presence of compound 7 (SEQ ID NO: 7) and compound 8 (SEQ ID NO: 8).

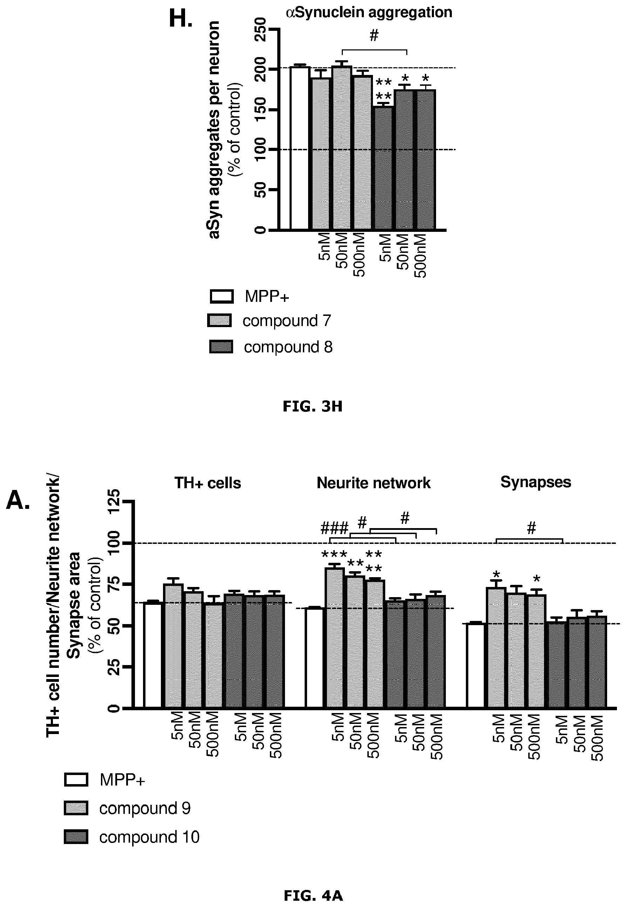

**FIGs. 4A-4H** show the neuroprotective effects of parent and retro-inverso compounds (compounds 9 - 16 having SEQ ID NO:s 9 - 16, respectively) on dopaminergic TH (tyrosine hydroxylase)-positive neurons injured with MPP+ (1-methyl-4-phenylpyridinium), and their effect on alpha-synuclein aggregation in TH-positive neurons. Data are expressed as a percentage of control non-injured condition as mean +/- SEM (n=4-6; MPP+ negative control n=122-127 collected across the multiple studies). * p<0.05, ** p<0.01, *** p<0.001, **** p<0.0001 Brown-Forsythe and Welch ANOVA test with post-hoc Dunnett pairwise comparison vs MPP+ negative control. # p<0.05, ## p<0.01, ### p<0.001, #### p<0.0001 Brown-Forsythe and Welch ANOVA test with post-hoc Dunnett pairwise comparison between parent and corresponding retro-inverso compound in the same concentration.
   **FIG. 4A** shows the number of TH neurons, total neurite network of TH neurons, and the number of synapses on TH neurites in a primary culture of mesencephalic cells after MPP+ injury in the presence of compound 9 (SEQ ID NO: 9) and compound 10 (SEQ ID NO: 10). Upper dashed line represents a control level of parameters (100%) obtained from non-injured cells; lower dashed line represents a negative control level of parameters obtained from cells injured with MPP+ without additional treatment with compounds.

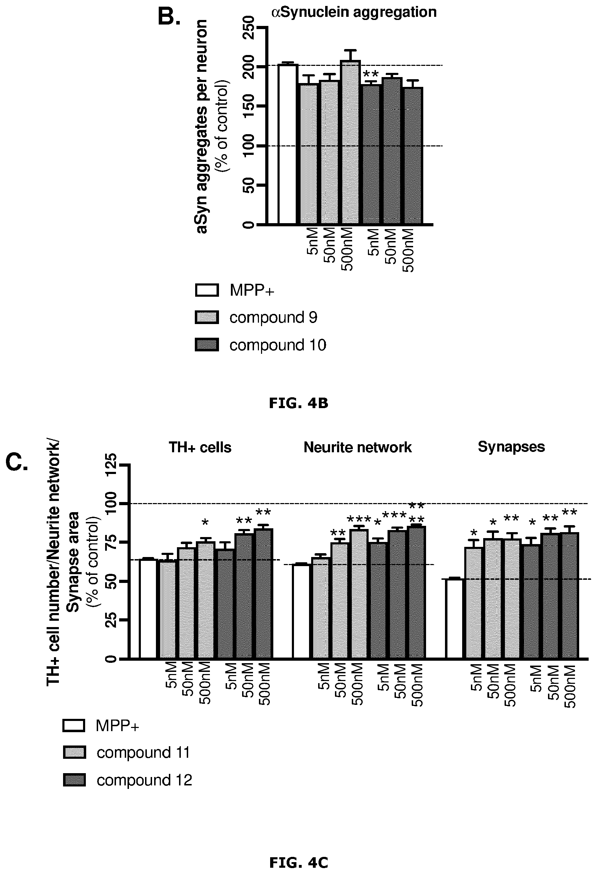

   **FIG. 4B** shows alpha-synuclein (aSyn) aggregation in TH neurons of a primary culture of mesencephalic cells after MPP+ injury in the presence of compound 9 (SEQ ID NO: 9) and compound 10 (SEQ ID NO: 10). Upper dashed line represents a negative control level of parameters obtained from cell injured with MPP+ without additional treatment with compounds; lower dashed line represents a negative control level of parameters (100%) obtained from non-injured cells.
   **FIG. 4C** shows the number of TH neurons, total neurite network of TH neurons, and the number of synapses on TH neurites in a primary culture of mesencephalic cells after MPP+ injury in the presence of compound 11 (SEQ ID NO: 11) and compound 12 (SEQ ID NO: 12).

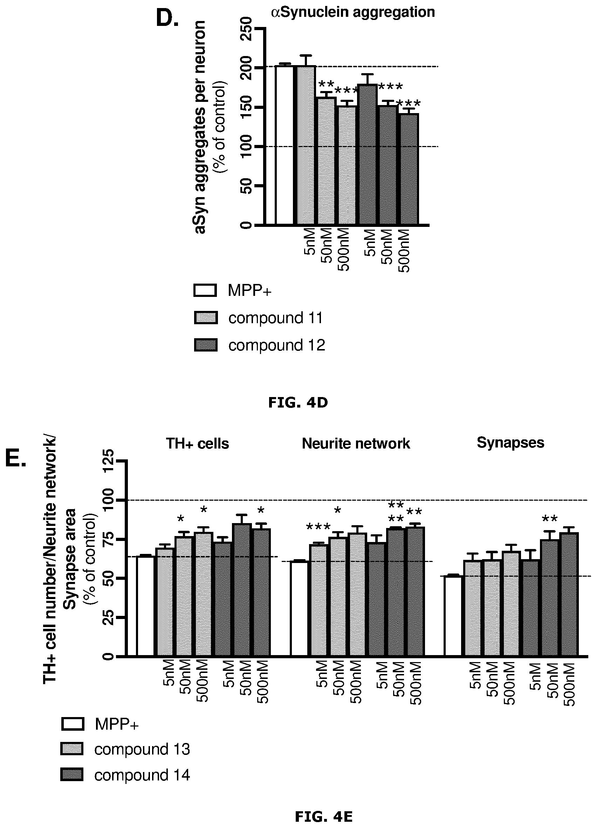

   **FIG. 4D** shows alpha-synuclein (aSyn) aggregation in TH neurons of a primary culture of mesencephalic cells after MPP+ injury in the presence of compound 11 (SEQ ID NO: 11) and compound 12 (SEQ ID NO: 12).
   **FIG. 4E** shows the number of TH neurons, total neurite network of TH neurons, and the number of synapses on TH neurites in a primary culture of mesencephalic cells after MPP+ injury in the presence of compound 13 (SEQ ID NO: 13) and compound 14 (SEQ ID NO: 14).

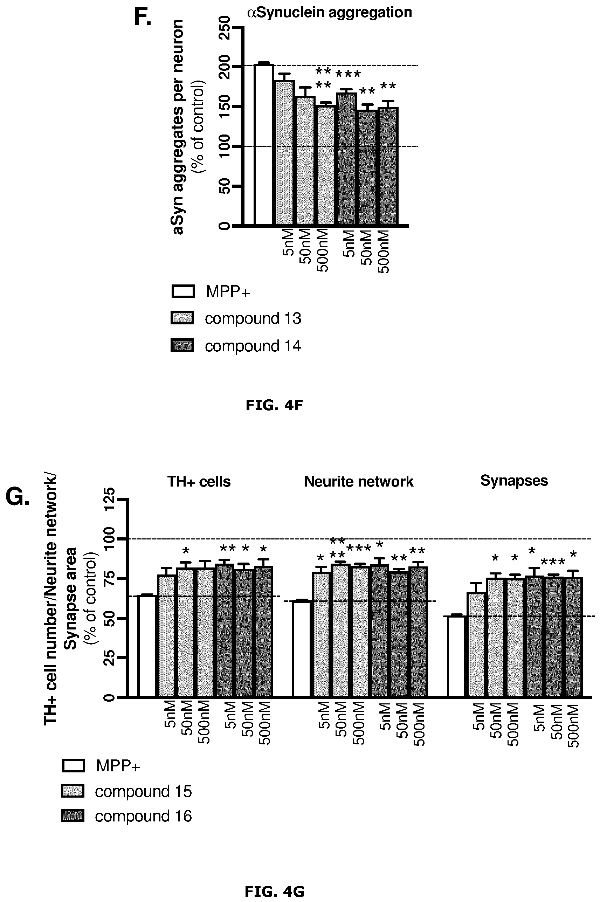

   **FIG. 4F** shows alpha-synuclein (aSyn) aggregation in TH neurons of a primary culture of mesencephalic cells after MPP+ injury in the presence of compound 13 (SEQ ID NO: 13) and compound 14 (SEQ ID NO: 14).
   **FIG. 4G** shows the number of TH neurons, total neurite network of TH neurons, and the number of synapses on TH neurites in a primary culture of mesencephalic cells after MPP+ injury in the presence of compound 15 (SEQ ID NO: 15) and compound 16 (SEQ ID NO: 16).

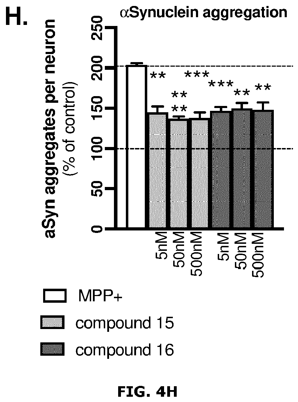

   **FIG. 4H** shows alpha-synuclein (aSyn) aggregation in TH neurons of a primary culture of mesencephalic cells after MPP+ injury in the presence of compound 15 (SEQ ID NO: 15) and compound 16 (SEQ ID NO: 16).

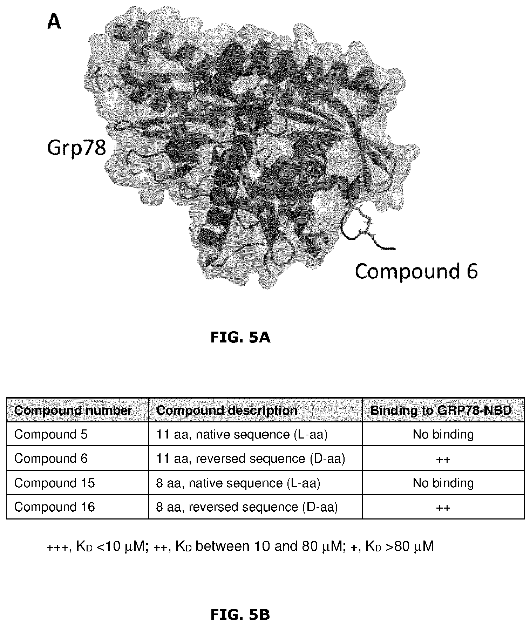

**FIG. 5A** shows the computational molecular model of the nucleotide-binding domain of GRP78 in complex with Compound 6. GRP78 is shown in a translucent surface model along with its cartoon trace. The Cys-Cys bond in compound 12 (SEQ ID NO: 6) is shown in sticks.
   **FIG. 5B** shows the microscale thermophoresis-based binding affinities of a representative set of peptides, compound 5 (SEQ ID NO: 5), compound 5 (SEQ ID NO: 6), compound 15 (SEQ ID NO: 15) and compound 16 (SEQ ID NO: 16) to GRP78 in a tabulated manner. Binding affinity is indicated as follows: +++, K_{D} <10 µM; ++, K_{D} 10-80 µM; +, K_{D} >80 µM, and no binding.

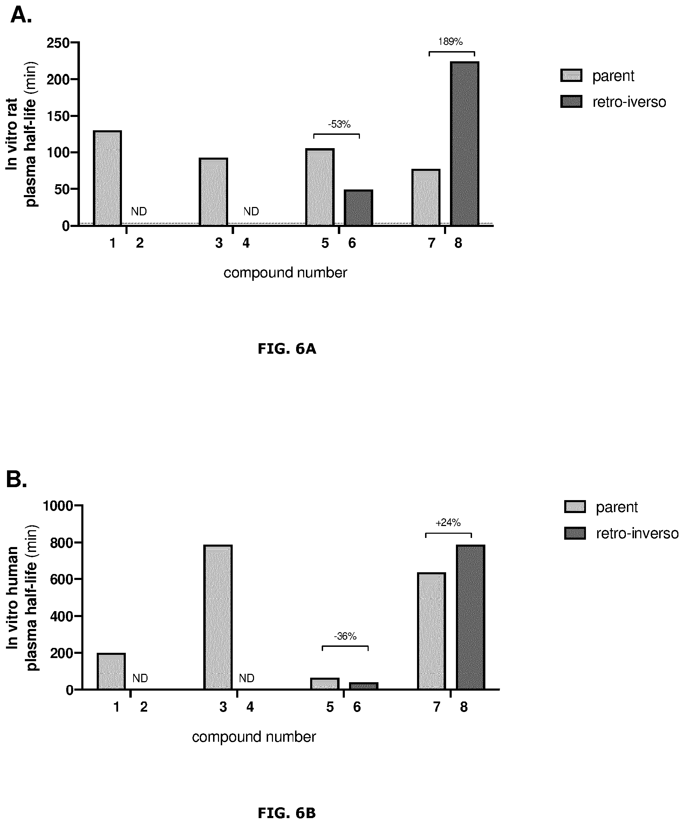

**FIGs. 6A-6B** show *in vitro* metabolic stability of parent and retro-inverso compounds (compounds 1 - 8 having SEQ ID NO:s 1 - 8, respectively) in rat and human plasma.
   **FIG. 6A** Calculated half-life based on compound disappearance in rat plasma.
   **FIG. 6B** Calculated half-life based on compound disappearance in human plasma. Arks and numbers above the columns reflect the change of half-life of retro-inverso compounds comparing to corresponding parent compounds. ND, not detected in the assay due to technical issues. The maximum calculated half-life at 789 min reflects experiment cut-off time limitation.

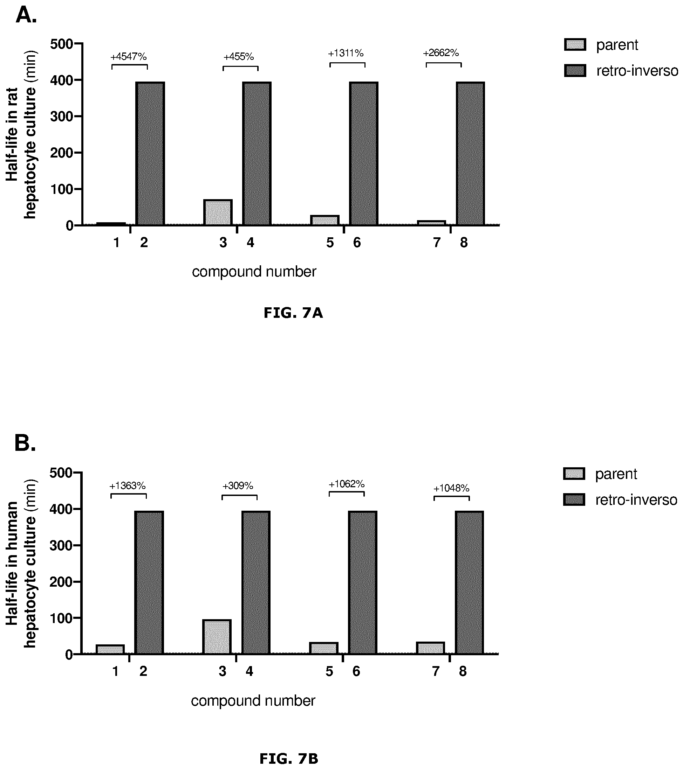

**FIGs. 7A-7B** show *in vitro* metabolic stability of parent and retro-inverso compounds (compounds 1 - 8 having SEQ ID NO:s 1 - 8, respectively) in rat and human hepatocytes.
   **FIG. 7A** Calculated half-life based on compound disappearance in rat liver hepatocytes.
   **FIG. 7B** Calculated half-life based on compound disappearance in human liver hepatocytes. Arks and numbers above the columns reflect the change of half-life of retro-inverso compounds as a percentage of corresponding parent compounds. The maximum calculated half-life at 395 min reflects experiment cut-off time limitation.

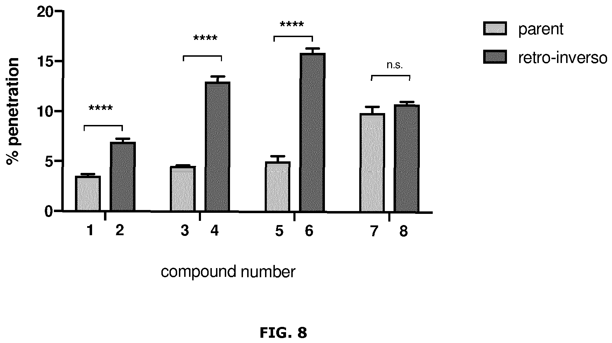

**FIG. 8** shows penetration of parent and retro-inverso compounds (compounds 1 - 8 having SEQ ID NO:s 1 - 8, respectively) through a 3D *in vitro* model of blood brain barrier. The amount of compound crossed the artificial blood brain barrier expressed in percentage of compound original applied concentration. Data are presented as mean +/- SEM (n=3-4). **** p<0.0001, n.s. not significant, Brown-Forsythe and Welch ANOVA test with post-hoc Dunnett pairwise comparison between parent and corresponding retro-inverso compound.

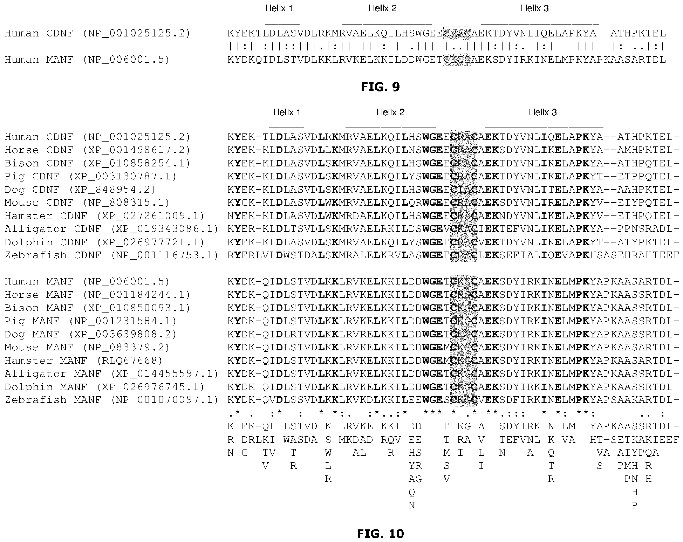

**FIG. 9****.** shows pairwise alignment of the C-terminal domains of CDNF and MANF. The alignment was performed using the following Genbank-retrieved sequences: for human CDNF accession # NP_001025125.2, and for human MANF accession # NP_006001.5. The CXXC motif is indicated gray background and the position of the three α-helices are shown.
**FIG. 10** shows ClustalW multiple sequence alignment of the C-terminal domains of CDNF and MANF (61-63 aa) from 10 different species (SEQ ID NO:s 43-62, respectively). The Genbank accession numbers are shown in the sequence alignment. The CXXC motif is indicated gray background and the position of the three α-helices are shown. Those residues conserved between these representative sequences (in both CDNF and MANF) are shown in bold. Below the sequence alignment, natural variants found in the representative 10 species per each position are shown. The presented list of sequences and species can be used to identify conserved and variable positions and shows that only limited variation is possible for most non-essential amino acid residues.

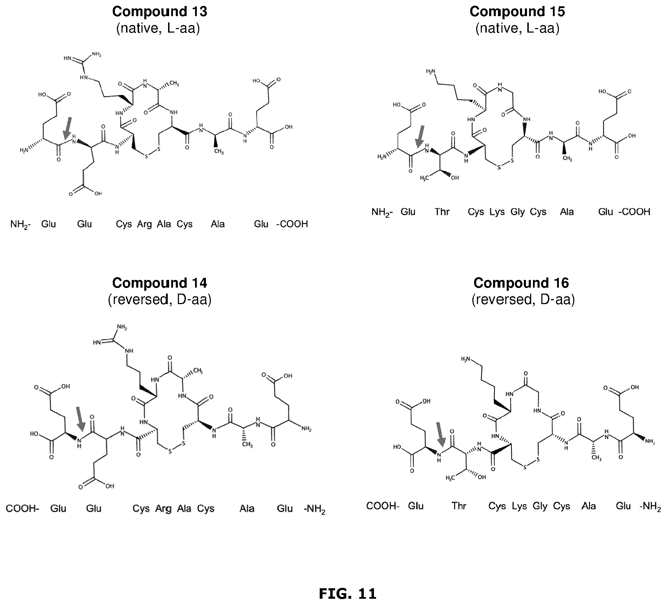

**FIG. 11** shows the structural formulas of Compounds 13-16 (SEQ ID NO:s 13-16, respectively). The amino acid names as standard abbreviations are shown below the formulas together with the sequence order (NH2 to COOH in native peptides and COOH to NH2 in retro-inverso peptides). The gray arrows point to single peptide bonds in each compound in order to illustrate the different order of amine and carbonyl groups in the amide peptide bond of linear peptides composed of L-amino acids and retro-inverso peptides composed of D-amino acids.

### SEQUENCE LISTING

**SEQ ID NO: 1** MRVAELKQILHSWGEECRACAEK
**SEQ ID NO: 2** KEACARCEEGWSHLIQKLEAVRM
**SEQ ID NO: 3** LRVKELKKILDDWGETCKGCAEK
**SEQ ID NO: 4** KEACGKCTEGWDDLIKKLEKVRL
**SEQ ID NO: 5** GEECRACAEKT
**SEQ ID NO: 6** TKEACARCEEG
**SEQ ID NO: 7** GETCKGCAEKS
**SEQ ID NO: 8** SKEACGKCTEG
**SEQ ID NO: 9** GEECRGACAEKT
**SEQ ID NO: 10** TKEACAGRCEEG
**SEQ ID NO: 11** GETCKGGCAEKS
**SEQ ID NO: 12** SKEACGGKCTEG
**SEQ ID NO: 13** EECRACAE
**SEQ ID NO: 14** EACARCEE
**SEQ ID NO: 15** ETCKGCAE
**SEQ ID NO: 16** EACGKCTE
**SEQ ID NO: 17** C-X₁-X₂-X₃-C
**SEQ ID NO: 18** E-X₄-C-X₁-X₂-X₃-C-A-E
**SEQ ID NO: 19** X₅-X₆-X₇-X₈-E-X₄-C-X₁-X₂-X₃-C-A-E-X₉-X₁₀-X₁₁ 
SEQ ID NO: 20 X12-X13-X14-X15-X16-X17-X18-X19-X20-X21-X22-X23-V-X24-E-L-K-X25-I-L-X5 X6-X7-X8-E-
X4-C-X1-X2-X3-C-A-E-X9-X10-X11
**SEQ ID NO: 21** RVAELKQILHSWGEECRACAEKTDYVNLIQELAPKYA, native human CDNF peptide
**SEQ ID NO: 22** RVKELKKILDDWGETCKGCAEKSDYIRKINELMPKYA, native human MANF peptide 
SEQ ID NO: 23 X16-X17-X18-X19-X20-X21-X22-X23-V-X24-E-L-K-X25-X26-L-X5-X6-X7-X8-E-X4-C-X1-X2-X3-
C-A-E-X9-X10-X11
 
SEQ ID NO: 24 X15-X16-X17-X18-X19-X20-X21-X22-X23-V-X24-E-L-K-X25-X26-L-X5-X6-X7-X8-E-X4-C-X1-
X2-X3-C-A-E-X9-X10
 
SEQ ID NO: 25 X14-X15-X16-X17-X18-X19-X20-X21-X22-X23-V-X24-E-L-K-X25-X26-L-X5-X6-X7-X8-E-X4-C-
X1-X2-X3-C-A-E-X9
 
SEQ ID NO: 26 X13-X14-X15-X16-X17-X18-X19-X20-X21-X22-X23-V-X24-E-L-K-X25-X26-L-X5-X6-X7-X8-E-X4-
C-X1-X2-X3-C-A-E
**SEQ ID NO: 27** KDEL
**SEQ ID NO: 28** CRAC
**SEQ ID NO: 29** CKGC
**SEQ ID NO: 30** CXXC
**SEQ ID NO: 31** ETCKGCAE
**SEQ ID NO: 32** TCKGCA 
SEQ ID NO: 33 MWCASPVAVV AFCAGLLVSH PVLTQGQEAG GRPGADCEVC KEFLNRFYKS
LIDRGVNFSL DTIEKELISF CLDTKGKENR LCYYLGATKD AATKILSEVT RPMSVHMPAM
KICEKLKKLD SQICELKYEK TLDLASVDLR KMRVAELKQI LHSWGEECRA CAEKTDYVNL
IQELAPKYAA THPKTEL full length CDNF (NCBI Reference Sequence: NP_001025125.2)
 
SEQ ID NO: 34 MRRMWATQGL AVALALSVLP GSRALRPGDC EVCISYLGRF YQDLKDRDVT
FSPATIENEL IKFCREARGK ENRLCYYIGA TDDAATKIIN EVSKPLAHHI PVEKICEKLK
KKDSQICELK YDKQIDLSTV DLKKLRVKEL KKILDDWGET CKGCAEKSDY IRKINELMPK
YAPKAASART DL full length MANF (NCBI Reference Sequence: NP_006001.5)
**SEQ ID NO: 35** TLDLASVDLRKMRVAELKQILHSWGEECRACAEKTDYVNLIQELAPKYA (49 aa CDNF)
**SEQ ID NO: 36** RVAELKQILHSWGEECRACAEKTDYVNLIQELAPKYA (37 aa CDNF)
**SEQ ID NO: 37** TLDLASVDLRKMRVAELKQILHSWGEECRACAEKT (35 aa CDNF)
**SEQ ID NO: 38** LASVDLRKMRVAELKQILHSWGEECRACAEKT (32 CDNF)
**SEQ ID NO: 39** QIDLSTVDLKKLRVKELKKILDDWGETCKGCAEKSDYIRKINELMPKYA (49 aa MANF)
**SEQ ID NO: 40** RVKELKKILDDWGETCKGCAEKSDYIRKINELMPKYA (37 aa MANF)
**SEQ ID NO: 41** QIDLSTVDLKKLRVKELKKILDDWGETCKGCAEKS (35 aa MANF)
**SEQ ID NO: 42** LSTVDLKKLRVKELKKILDDWGETCKGCAEKS (32 aa MANF)

SEQ ID NO: 43 KYEKTLDLASVDLRKMRVAELKQILHSWGEECRACAEKTDYVNLIQELAPKYA
ATHPKTEL, Human CDNF (NP_001025125.2)

SEQ ID NO: 44 KYEK-KLDLASVDLLKMRVAELKQILNSWGEECRACAEKSDYVNLIKELAPKYA
AMHPKTEL, Horse CDNF (XP_001498617.2)

SEQ ID NO: 45 KYEKKLDLASVDLSKMRVAELKQILHGWGEECRACAEKTDYVNLIKELAPKYA
ATHPQTEL, Bison CDNF (XP_010858254.1)

SEQ ID NO: 46 KYEK-KLDLASVDLSKMRVAELQILYSWGEECRACAEKTDYVNLIKELAPKYTE
TPPQTEL, Pig CDNF (XP_003130787.1)

SEQ ID NO: 47 KYEK-KLDLASVDLSKMRVAELKQILHSWGEECIACAEKTDYVNLITELAPKYAA
AHPKTEL, Dog CDNF (XP_848954.2)

SEQ ID NO: 48 KYGK-KLDLASVDLWKMRVAELKQILQRWGEECRACAEKSDYVNLIRELAPKY
VEIYPQTEL, Mouse CDNF (NP_808315.1)

SEQ ID NO: 49 NYEK-KLDLASVDLWKMRDAELKQILHSWGEECRACAEKNDYVNLIKELAPKY
VEIHPQIEL, Hamster CDNF (XP_027261009.1)

SEQ ID NO: 50 KYER-KLDLTSVDLSKMRVAELRKILDSWGEVCKACIEKTEFVNLIKELAPKYA
PPNSRADL, Alligator CDNF (XP_019343086.1)

SEQ ID NO: 51 KYEK-KLDLASVDLSKMRVAELKQILYSWGEECRACVEKTDYVNLIKELAPKYT
ATYPKTEL, Dolphin CDNF (XP_026977721.1)

SEQ ID NO: 52 RYERLVLDWSTDALSKMRALELKRVLASWGEECRACLEKSEFIALIQEVAPKH
SASEHRAHTEEF, Zebrafish CDNF (NP_001116753.1)

SEQ ID NO: 53 KYDK-QIDLSTVDLKKLRVKELKKILDDWGETCKGCAEKSDYIRKINELMPKYAP
KAASARTDL, Human MANF (NP_006001.5)

SEQ ID NO: 54 KYDK-QIDLSTVDLKKLRVKELKKILDDWGETCKGCAEKSDYIRKINELMPKYAP
KAASSRTDL, Horse MANF (NP_001184244.1)

SEQ ID NO: 55 KYDK-QIDLSTVDLKKLRVKELKKILDDWGETCKGCAEKSDYIRKINELMPKYAP
KAASSRTDL, Bison MANF (XP_010850093.1)

SEQ ID NO: 56 KYDK-QIDLSTVDLKKLRVKELKKILDDWGETCKGCAEKSDYIRKINELMPKYAP
KAASSRTDL, Pig MANF (NP_001231584.1)

SEQ ID NO: 57 KYDK-QIDLRTVDLKKLRVRELKKILDDWGETCKGCAEKSDYIRKINELMPKYAP
KAASSRTDL, Dog MANF (XP_003639808.2)

SEQ ID NO: 58 KYDK-QIDLSTVDLKKLRVKELKKILDDWGEMCKGCAEKSDYIRKINELMPKYAP
KAASARTDL, Mouse MANF (NP_083379.2)

SEQ ID NO: 59 KYDK-QIDLSTVDLKKLRVKELKKILDDWGEMCKGCAEKSDYIRKINELMPKYAP
KAASARTDL, Hamster MANF (RLQ67668)

SEQ ID NO: 60 KYDK-QIDLSTVDLKKLRVKELKKILDDWGETCKGCAEKSDYIRKINELMPKYAP
KAASSRTDL, Alligator MANF (XP_014455597.1)

SEQ ID NO: 61 KYDK-QIDLSTVDLKKLRVKELKKILDDWGETCKGCAEKSDYIRKINELMPKYAP
KAASSRTDL, Dolphin MANF (XP_026976745.1)

SEQ ID NO: 62 KYDK-QVDLSSVDLKKLKVKDLKKILEEWGESCKGCVEKSDFIRKINELMPKYA
PSAAKARTDL, Zebrafish MANF (NP_001070097.1)

### DETAILED DESCRIPTION OF THE INVENTION

The term "modified peptide" refers to a peptide or polypeptide, which has been modified or synthesized. Peptide modification or synthesis options include e.g. retro-inverso isomerized peptides, cyclic peptides, peptidomimetics, click chemistry, stapled peptides, N-terminal modifications, C-terminal modifications, isotope labeled peptides, biotinylated and tagged peptides, fluorescent dye labeled peptides, peptide dimers, post-translational modifications, internally quenched/FRET peptides, linker/spacer/PEGylations, peptide pooling, protein conjugation, immunogenic peptides, and incorporation of unnatural amino acids. A "non-naturally encoded amino acid" refers to an amino acid that is not one of the 20 common amino acids or pyrrolysine or selenocysteine. Other terms that may be used synonymously with the term "non-naturally encoded amino acid" are "non-natural amino acid," "unnatural amino acid," "non-naturally-occurring amino acid," and variously hyphenated and non-hyphenated versions thereof. The term "non-naturally encoded amino acid" also includes, but is not limited to, [amino acids that occur by modification (e.g. post-translational modifications) of a naturally encoded amino acid (including but not limited to, the 20 common amino acids or pyrrolysine and selenocysteine) but are not themselves naturally incorporated into a growing polypeptide chain by the translation complex. Examples of such non-naturally-occurring amino acids include, but are not limited to, N-acetylglucosaminyl-L-serine, N-acetylglucosaminyl-L-threonine, and O-phosphotyrosine]

The term "retro-inverso" refers to a peptide sequence wherein one or more of the amino acids are D amino acids (inverso) and the peptide sequence is in the reverse order (retro). In some embodiments there may be 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, or 32 D amino acids. In some embodiments, the retro-inverso peptides include amino acids with alternating chirality. In certain embodiments, the retro-inverso peptides include all D amino acids. As used herein, chirality refers to the D and L isomers of amino acids. To illustrate, a retro-inverso peptide having the sequence CX₁X₂X₃C would be inversed to have the sequence CX₃X₂X₁C, wherein at least one or more amino acids is D amino acids. Peptides made of D-amino acids are metabolically stable as they are poor substrates for proteases which have evolved to degrade proteins and peptides made of L-amino acids. However, D-peptides have reversed handedness in e.g. helical structures, i.e. the amino acid side chains are positioned as a mirror image. Reversing the sequence order in D-peptides provides a structure that mimics the L-peptide analog in side chain orientation, in other words retro-inverso isomerization. Short non-helical retro-inverso peptides can functionally mimic their natural L-protein counterparts in target binding.

The principle of retro-inverso isomerization is that e.g. when linear peptide is NH2- Gly – L-Glu – L-Glu – L-Cys – L-Arg – L-Ala – L-Cys – L-Ala – L-Glu – L-Lys – L-Thr - COOH (SEQ ID NO: 5) the retro-inverso isomerized form is

NH2- D-Thr – D-Lys – D-Glu – D-Ala – D-Cys – D-Ala – D-Arg – D-Cys – D-Glu – D-Glu – Gly -
COOH (SEQ ID NO: 6)

An example of retro-inverso isomerization principle is also presented in Fig. 11 for Compounds 13 - 16 (SEQ ID NOs: 13 - 16, respectively).

The term "pseudopeptide" refers to an amide of an amino acid that does not occur in natural peptides or proteins, especially one introduced into a polypeptide chain. Pseudopeptides or amino bond surrogates are among a variety of terms that can be used to describe backbone-modified peptides. These synthetic analogs of peptides have a variety of potential uses, but most of the expanded interest in these areas focuses on their potential for developing metabolically stabilized and perhaps orally active peptide hormone analogs or enzyme inhibitors with enhanced biological potency. The term specifically includes peptide back-bone modifications (i.e., amide bond mimetics) known to those skilled in the art. Such modifications include modifications of the amide nitrogen, the α-carbon, amide carbonyl, complete replacement of the amide bond, extensions, deletions or backbone crosslinks. Several peptide backbone modifications are known, including ψ[CH2S], ψ[CH2NH], ψ[CSNH2], ψ[NHCO], ψ[COCH2], and ψ[(E) or (Z) CH=CH]. In the nomenclature used above, ψ indicates the absence of an amide bond. The structure that replaces the amide group is specified within the brackets.

As used herein, when two entities are "conjugated" to one another they are linked by a direct or indirect covalent or non-covalent interaction. In certain aspects, the association is covalent. In other aspects, the association is non-covalent. Non-covalent interactions include hydrogen bonding, van der Waals interactions, hydrophobic interactions, magnetic interactions, electrostatic interactions, etc. An indirect covalent interaction is when two entities are covalently connected, optionally through a linker group. "Conjugation" means herein that a peptide conjugated or coupled to a detectable chemical or biochemical moiety, or PEG or other moieties that are used to prolong plasma half-life. In some instances, one or more peptides disclosed herein can be conjugated, for example, to a carrier protein. Such conjugated compositions can be monovalent or multivalent. For example, conjugated compositions can include one peptide disclosed herein conjugated to a carrier protein. Alternatively, conjugated compositions can include two or more peptides disclosed herein conjugated to a carrier.

The "blood-brain barrier" or "BBB" is a highly selective semipermeable membrane barrier that separates the circulating blood from the brain and extracellular fluid in the central nervous system. The BBB is formed is formed by endothelial cells of the capillary wall, astrocyte end-feet ensheathing the capillary, and pericytes embedded in the capillary basement membrane. The system allows the passage of some molecules by passive diffusion, as well as the selective transport of molecules such as glucose, water and amino acids that are crucial to neural function. Large molecules such as proteins typically cannot pass through the BBB. However, some peptides can cross the BBB through various mechanisms, and also some proteins, that contain specific recognition motifs for transporter proteins residing at the surface of brain vascular endothelial cells, can get transported across the BBB.

As used herein, "pharmaceutically acceptable carrier" may include one or more solvents, buffers, solutions, dispersion media, coatings, antibacterial and antifungal agents, isotonic and absorption delaying agents and the like acceptable for use in formulating pharmaceuticals, such as pharmaceuticals suitable for administration to humans. The use of such media and agents for pharmaceutically active substances is well known in the art. Supplementary active ingredients also can be incorporated into the compositions.

CDNF and MANF share ca. 60% amino acid sequence homology (Figs. 9 and 10), but they have highly similar three-dimensional structures. Both CDNF and MANF are composed of two independently folded domains connected by a flexible loop region. The secondary structure is predominantly α-helical, with five α-helices in the N-terminal domain, and three α-helices in the C-terminal domain. Three disulphide bridges stabilize the N-terminal domain while the C-terminal CRAC (SEQ ID NO: 28) sequence, CKGC (SEQ ID NO: 29) in MANF, forms an internal disulphide bridge. This CXXC (SEQ ID NO: 30) disulphide bridge is found both in CDNF and MANF. The CXXC motif is beneficial for the neuroprotective activity of MANF and CDNF. However, the data presented here show that the CXXC (SEQ ID NO: 30) motif can accommodate some modifications, such as addition of a small amino acid (e.g. glycine and serine), i.e. specific types of CXXXC motifs can also be used. In some aspects, CDNF has a sequence derived from NP_001025125.2 (SEQ ID NO: 33). In some aspects, MANF has a sequence derived from NP_006001.5 (SEQ ID NO: 34.

In addition to naturally occurring allelic variants derived from MANF and CDNF peptides, changes can be introduced by mutation into MANF/CDNF sequences that incur alterations in the amino acid sequences of the encoded MANF/CDNF peptide. Nucleotide substitutions leading to amino acid substitutions at "non-essential" amino acid residues can be made in the sequence of a MANF/CDNF peptide. MANF/CDNF peptides or functional fragments thereof comprising one or more "non-essential" substitutions can be seen as equivalents to wild-type MANF/CDNF peptides disclosed herein.

Each amino acid can be a natural or non-natural amino acid. The term "non-natural amino acid" refers to an organic compound that is a congener of a natural amino acid in that it has a structure similar to a natural amino acid so that it mimics the structure and reactivity of a natural amino acid. The non-natural amino acid can be a modified amino acid, and/or amino acid analog, that is not one of the 20 common naturally occurring amino acids or the rare natural amino acids selenocysteine or pyrolysine. Non-natural amino acids can also be the D-isomer of the natural amino acids.

Examples of suitable amino acids include, but are not limited to, alanine, alloisoleucine, arginine, asparagine, aspartic acid, cysteine, cyclohexylalanine, 2,3-diaminopropionic acid, 4-fluorophenylalanine, glutamine, glutamic acid, glycine, histidine, homoproline, isoleucine, leucine, lysine, methionine, naphthylalanine, norleucine, phenylalanine, phenylglycine, pipecolic acid, proline, pyroglutamic acid, sarcosine, serine, selenocysteine, threonine, tryptophan, tyrosine, valine, a derivative, or combinations thereof.

The term "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

A "pharmaceutically acceptable salt" is intended to mean a salt of a free acid or base of a compound represented herein that is non-toxic, biologically tolerable, or otherwise biologically suitable for administration to the subject. See, generally, S.M. Berge, et al., "Pharmaceutical Salts," J. Pharm. Sci., 1977, 66, 1-19. Preferred pharmaceutically acceptable salts are those that are pharmacologically effective and suitable for contact with the tissues of subjects without undue toxicity, irritation, or allergic response. A compound described herein may possess a sufficiently acidic group, a sufficiently basic group, both types of functional groups, or more than one of each type, and accordingly react with a number of inorganic or organic bases, and inorganic and organic acids, to form a pharmaceutically acceptable salt.

For a compound described herein that contains a basic group, such as an amine, a pharmaceutically acceptable salt may be prepared by any suitable method available in the art, e.g., treatment of the free base with an inorganic acid, such as hydrochloric acid, hydrobromic acid, sulfuric acid, sulfamic acid, nitric acid, boric acid, phosphoric acid, and the like, or with an organic acid, such as acetic acid, phenylacetic acid, propionic acid, stearic acid, lactic acid, ascorbic acid, maleic acid, hydroxymaleic acid, isethionic acid, succinic acid, valeric acid, fumaric acid, malonic acid, pyruvic acid, oxalic acid, glycolic acid, salicylic acid, oleic acid, palmitic acid, lauric acid, a pyranosidyl acid, such as glucuronic acid or galacturonic acid, an alpha-hydroxy acid, such as mandelic acid, citric acid, or tartaric acid, an amino acid, such as aspartic acid or glutamic acid, an aromatic acid, such as benzoic acid, 2-acetoxybenzoic acid, naphthoic acid, or cinnamic acid, a sulfonic acid, such as laurylsulfonic acid, p-toluenesulfonic acid, methanesulfonic acid, or ethanesulfonic acid, or any compatible mixture of acids such as those given as examples herein, and any other acid and mixture thereof that are regarded as equivalents or acceptable substitutes in light of the ordinary level of skill in this technology.

For a compound described herein that contains an acidic group, such as a carboxylic acid group, base addition salts can be prepared by any suitable method available in the art, e.g., treatment of such compound with a sufficient amount of the desired the desired base, either neat or in a suitable inert solvent. Examples of pharmaceutically acceptable base addition salts include, but are not limited to, lithium, sodium, potassium, calcium, ammonium, zinc, or magnesium salt, or other metal salts; organic amino salts, such as, alkyl, dialkyl, trialkyl, or tetra-alkyl ammonium salts.

Other examples of pharmaceutically acceptable salts include, but are not limited to, camsylate, sulfates, pyrosulfates, bisulfates, sulfites, bisulfites, phosphates, monohydrogen-phosphates, dihydrogenphosphates, metaphosphates, pyrophosphates, chlorides, bromides, iodides, acetates, propionates, decanoates, caprylates, acrylates, formates, isobutyrates, caproates, heptanoates, propiolates, oxalates, malonates, succinates, suberates, sebacates, fumarates, maleates, butyne-1,4-dioates, hexyne-1,6-dioates, benzoates, chlorobenzoates, methylbenzoates, dinitrobenzoates, hydroxybenzoates, methoxybenzoates, phthalates, sulfonates, methylsulfonates, propylsulfonates, besylates, xylenesulfonates, naphthalene-1-sulfonates, naphthalene-2-sulfonates, phenylacetates, phenylpropionates, phenylbutyrates, citrates, lactates, γ-hydroxybutyrates, glycolates, tartrates, and mandelates. Lists of other suitable pharmaceutically acceptable salts are found in Remington's Pharmaceutical Sciences, 17th Edition, Mack Publishing Company, Easton, Pa., 1985.

The neutral forms of the compounds are preferably regenerated by contacting the salt with a base or acid and isolating the parent compound in the conventional manner. The parent form of the compound differs from the various salt forms in certain physical properties, such as solubility in polar solvents, but otherwise the salts are equivalent to the parent form of the compound for the purposes of the present application.

In the embodiments of the disclosure, the length of the peptide or fragment is in the range of 8 - 32 amino acids, wherein the peptide or fragment thereof comprises CX₁X₂X₃C (SEQ ID NO: 17) as described herein. In certain embodiments, the preferred peptides or fragments can consist of 8, 9, 10, 11, 12, 13, 14, 15, 16, 17, 18, 19, 20, 21, 22, 23, 24, 25, 26, 27, 28, 29, 30, 31, or 32 amino acids. In certain embodiments, the length of the peptide or fragment is in the range of 8 - 23, 11 - 23, 12 - 23, 8 - 11 or 8 - 12 amino acids. In certain embodiments, the length of the peptide or fragment is in the range of 8-31, 8-29, 8-27, 8-25, 8-23, 8-21, 8-19, 8-17, 8-15, 11-27, 11-25, 11-23, 11-21, 11-19, 11-17, 11-15, 12-25, 12-23, 12-21, 12-19, 12-17, 12-15, 23-27, 24-27, or 25-27 amino acids. The fragments may comprise any of the naturally occurring amino acids such as alanine [Ala (A)], arginine [Arg (R)], asparagine [Asn (N)], aspartic acid [Asp (D)], cysteine [Cys (C)], glutamine [Gln (Q)], glutamic acid [Glu (E))], glycine [Gly (G)], histidine [His (H)], isoleucine [Ile (I)], leucine [Leu (L)], lysine (Lys (K)], methionine [Met (M)], phenylalanine Phe (F)], proline [Pro (P)], serine [Ser (S)], threonine [Thr (T)], tryptophan [Trp (W)], tyrosine [Tyr (Y)], and valine [Val (V)] as well as non-natural or modified amino acids.

The present disclosure provides a peptide having a length of 8 - 32 amino acids or a pharmaceutically acceptable salt thereof comprising a retro-inverso form of an amino acid sequence of C-X₁-X₂-X₃-C (SEQ ID NO: 17),
wherein
X₁ is selected from the group consisting of R, K, I, G, A and S;
X₂ is absent or selected from the group consisting of G, A, R, K, I and S; and
X₃ is selected from the group consisting of A, G and S.

In a preferred embodiment the peptide comprises a retro-inverso form of an amino acid sequence of E-X₄-C-X₁-X₂-X₃-C-A-E (SEQ ID NO:18),
wherein
X₁ is selected from the group consisting of R, K, I, G, A and S;
X₂ is absent or selected from the group consisting of G, A, R, K, I, and S;
X₃ is selected from the group consisting of A, G and S; and
X₄ is selected from the group consisting of E, T, V, D, M and G.

Based on the natural variation in CDNF and MANF sequences in different species (human, horse, bison, pig, dog, mouse, hamster, alligator, dolphin and zebrafish CDNF and MANF are used as example sequences in Fig. 10), limited changes in the peptide sequence regarding X-groups compared to the human sequences can be accommodated without losing biological activity.

In another preferred embodiment the peptide comprises a retro-inverso form of an amino sequence of X₅-X₆-X₇-X₈-E-X₄-C-X₁-X₂-X₃-C-A-E-X₉-X₁₀-X₁₁ (SEQ ID NO: 19),
wherein
X₁ is selected from the group consisting of R, K, I, G, A, and S;
X₂ is absent or selected from the group consisting of G, A, R, K, I and S;
X₃ is selected from the group consisting of A, G and S;
X₄ is selected from the group consisting of E, T, V, D, M and G;
X₅ is absent or selected from the group consisting of H, D, Q, R, Y, N and S;
X₆ is absent or selected from the group consisting of S, D, G, N and R;
X₇ is absent or W;
X₈ is absent or G;
X₉ is absent or K;
X₁₀ is absent or selected from the group consisting of T, S, A, I and N; and
X₁₁ is absent or selected from D and E.

In another preferred embodiment the peptide comprises a retro-inverso form of an amino sequence, which is within an amino acid sequence of X₁₂-X₁₃-X₁₄-X₁₅-X₁₆-X₁₇-X₁₈-X₁₉-X₂₀-X₂₁-X₂₂-X₂₃-V-X₂₄-E-L-K-X₂₅-X₂₆-L-X₅-X₆-X₇-X₈-E-X₄-C-X₁-X₂-X₃-C-A-E-X₉-X₁₀-X₁₁ (SEQ ID NO: 20),
wherein
X₁ is selected from the group consisting of R, K, I, G, A and S;
X₂ is absent or selected from the group consisting of G, A, R, K, I and S;
X₃ is selected from the group consisting of A, G and S;
X₂ is absent or selected from the group consisting of G, A, R, K, I and S;
X₃ is selected from the group consisting of A, G and S;
X₄ is selected from the group consisting of E, T, V, D, M and G;
X₅ is absent or selected from the group consisting of H, D, Q, R, Y, N and S;
X₆ is absent or selected from the group consisting of S, D, G, N and R;
X₇ is absent or W;
X₈ is absent or G;
X₉ is absent or K;
X₁₀ is absent or selected from the group consisting of T, S, A, I and N; and
X₁₁ is absent or selected from D and E,
X₁₂ is absent or selected from the group consisting of L, I and V;
X₁₃ is absent or D;
X₁₄ is absent or selected from L and W;
X₁₅ is absent or selected from the group consisting of A, S, T, E and N;
X₁₆ is absent or selected from S and T;
X₁₇ is absent or selected from V and D;
X₁₈ is absent or selected from D and A;
X₁₉ is absent or L;
X₂₀ is absent or selected from the group consisting of R, K, S and W;
X₂₁ is absent or K;
X₂₂ is absent or selected from the group consisting of M, L, I and V;
X₂₃ is absent or R;
X₂₄ is selected from the group consisting of A, K, T, L and V;
X₂₅ is selected from the group consisting of Q, K and R; and
X₂₆ is selected from I and V.

In some embodiments, the peptide comprises an 8-23 amino acid long peptide within SEQ ID NO:20, wherein the peptide includes the CX₁X₂X₃C (SEQ ID NO: 17) motif.

In another preferred embodiment the peptide comprises a retro-inverso form of an amino sequence, which is within an amino acid sequence selected from the group consisting of: 
X16-X17-X18-X19-X20-X21-X22-X23-V-X24-E-L-K-X25-X26-L-X5-X6-X7-X8-E-X4-C-X1-X2-X3-C-A-E-X9-X10-
X11 (SEQ ID NO: 31),
 
X15-X16-X17-X18-X19-X20-X21-X22-X23-V-X24-E-L-K-X25-X26-L-X5-X6-X7-X8-E-X4-C-X1-X2-X3-C-A-E-X9-
X10 (SEQ ID NO: 32),
 
X14-X15-X16-X17-X18-X19-X20-X21-X22-X23-V-X24-E-L-K-X25-X26-L-X5-X6-X7-X8-E-X4-C-X1-X2-X3-C-A-E-
X9 (SEQ ID NO: 33),
 
X13-X14-X15-X16-X17-X18-X19-X20-X21-X22-X23-V-X24-E-L-K-X25-X26-L-X5-X6-X7-X8-E-X4-C-X1-X2-X3-C-A-
E (SEQ ID NO: 34).
 and

In some embodiments, the peptide comprises an 8-23 amino acid long peptide within any one of SEQ ID NOs: 31-34, wherein the peptide includes the CX₁X₂X₃C (SEQ ID NO: 17) motif.
In certain instances, the peptide comprises an amino acid sequence which may be within an amino acid sequence SEQ ID NO: 35 or within an amino acid sequence SEQ ID NO: 39.

Preferably, the peptide consists of a sequence selected from the group consisting of: KEACARCEEGWSHLIQKLEAVRM (SEQ ID NO: 2), KEACGKCTEGWDDLIKKLEKVRL (SEQ ID NO: 4), TKEACARCEEG (SEQ ID NO: 6), SKEACGKCTEG (SEQ ID NO: 8), TKEACAGRCEEG (SEQ ID NO: 10), SKEACGGKCTEG (SEQ ID NO: 12), EACARCEE (SEQ ID NO: 14), and EACGKCTE (SEQ ID NO: 16), wherein all amino acids of the peptide are D-amino acids.

In some embodiments, the N-terminus of the peptide is acetylated. In some embodiments, the C-terminus of the peptide is amidated. In some embodiments, the N-terminus of the peptide is acetylated and the C-terminus of the peptide is amidated.

In some embodiments, the peptide is a pseudopeptide.
In an embodiment the peptide is cyclic.

In certain instances, the peptide is 11-32 amino acids in length. In certain instances, the peptide is 12-32 amino acids in length. In certain instances, the peptide is 12-23 amino acids in length. In certain instances, the peptide is 8-23 amino acids in length. In certain instances, the peptide is 8-13 amino acids in length. In certain instances, the peptide is 8-12 amino acids in length.

In a preferred embodiment in the modified peptide cysteine (C) is in a reduced form or in disulphide bridged form.

In certain embodiments, the peptide described herein binds to GRP78.

In some aspects, the peptide described herein is 1.5-fold more stable than its parent counterpart. In some aspects, the peptide described has a half-life that is 1.5-fold higher than its parent counterpart.

In some aspects, the peptide may comprise a linkage connecting the N-terminus to the C-terminus of the peptide.

In some aspects, the N-terminus of the peptide may be acetylated. In some aspects, the C-terminus of the peptide is amidated. In some aspects the N-terminus of the peptide may be acetylated and the C-terminus of the peptide may be amidated.

In another preferred embodiment the peptide is conjugated to a detectable moiety, chemical moiety, or biochemical moiety, or polyethylene glycol (PEG).

The peptide may be conjugated to a detectable chemical or biochemical moiety such as a fluorophore (e.g. fluorescein or rhodamine). Radiolabeling of the peptide may be used, e.g.for use in SPECT or PET imaging. As used herein, a "detectable chemical or biochemical moiety" means a chemical tag that exhibits an amino acid sequence or a detectable chemical or biochemical moiety for the purpose of facilitating detection of the peptide; such as a detectable molecule selected from among: a visible, fluorescent, chemiluminescent, or other detectable chemical tag; an enzyme that is detectable in the presence of a substrate, e.g., an alkaline phosphatase with NBT plus BCIP or a peroxidase with a suitable substrate; a detectable protein, e.g., a green fluorescent protein. Preferably, the tag does not prevent or hinder the penetration of the fragment into a target cell or otherwise alter the biological activity of the compound.

N- and/or C-terminal modifications of the C-terminal CDNF fragments or C-terminal MANF fragments to further increase the stability and/or cell permeability of the peptides or fragments are also preferred. Acetylation - amidation of the termini of the CDNF fragment or MANF fragment (i.e. N-terminal acetylation and C-terminal amidation) is one of the options known in the art (see e.g. Marino et al. 2015, ACS Chem. Biol. 10: 1754-1764).

In some instances, the peptide as described herein has at least one (e.g., 1, 2, 3, 4, 5, 6, or 7) of the following properties: (i) the peptide can dose-dependently protect TH-positive neurons from MPP+ toxicity; (ii) the peptide reduces the number of a-synuclein inclusions in TH-positive neurons; (iii) the peptide has improved stability in plasma compared to its parent counterpart; (iv) the peptide has improved stability in hepatocytes compared to its parent counterpart; or (v) the peptide has improved ability to pass through the blood brain barrier compared to its parent counterpart.

An embodiment provides the peptide as described herein for use as a medicament.

Since CDNF/MANF peptides potently protected the dopamine neurons from death the prior art such as WO2009133247, and EP 1969003 shows that the peptides can be used in the treatment of central nervous system (CNS) diseases such as Alzheimer's disease, Parkinson's disease (PD), multiple system atrophy, amyotrophic lateral sclerosis (ALS), frontotemporal lobar degeneration, dementia with Lewy bodies, mild cognitive impairment, Huntington's disease (HD), traumatic brain injury, drug addiction and stroke.

CDNF and MANF modulate signaling of the unfolded protein response (UPR) pathway and protect cells from ER stress-related cell death. ER stress is known to play an important pathophysiological role in diverse chronic diseases, such as neurodegenerative and metabolic diseases and acute injuries (Wang and Kaufman, 2016). GRP78 (a.k.a. BiP and HSPA5) is a major ER lumenal chaperone and a master regulator of the UPR (Bertolotti et al, 2000; Wang and Kaufman, 2016). Dynamic association and dissociation of GRP78 with UPR receptors IRE1α, PERK and ATF6 is a key step regulating the signaling activity of the UPR receptors under ER stress. Interaction of MANF with GRP78 regulates its cellular activities (Yan et al, 2019).

Accordingly, the present disclosure is directed to a method for treatment of a degenerative, chronic, or progressive disease or disorder, such as a CNS disease or disorder, or a monogenic hereditary disease (having ER stress as a pathogenic component), wherein a pharmaceutically effective amount of the peptide with the length of 8 - 32 amino acids comprising the sequence C-X₁-X₂-X₃-C (SEQ ID NO:17), E-X₄-C-X₁-X₂-X₃-C-A-E (SEQ ID NO:18), X₅-X₆-X₇-X₈-E-X₄-C-X₁-X₂-X₃-C-A-E-X₉-X₁₀-X₁₁ (SEQ ID NO: 19) or X₁₂-X₁₃-X₁₄-X₁₅-X₁₆-X₁₇-X₁₈-X₁₉-X₂₀-X₂₁-X₂₂-X₂₃-V-X₂₄-E-L-K-X₂₅-X₂₆-L-X₅-X₆-X₇-X₈-E-X₄-C-X₁-X₂-X₃-C-A-E-X₉-X₁₀-X₁₁ (SEQ ID NO: 20), wherein said peptide is a retro-inverso form of said amino acid sequence, is administered to a patient.

Another embodiment provides the peptide for use in the treatment of a degenerative, chronic, or progressive disease or disorder, such as a neurodegenerative disease or disorder.
Said neurodegenerative disease or disorder is preferably a central nervous system disease selected from the group consisting of: Parkinson's disease, Alzheimer's disease, multiple system atrophy, amyotrophic lateral sclerosis, frontotemporal lobar degeneration, dementia with Lewy bodies, mild cognitive impairment, Huntington's disease, traumatic brain injury, traumatic spinal cord injury, progressive supranuclear palsy, Pick's disease, pure autonomic failure, corticobasal degeneration, chronic traumatic encephalopathy, spinocerebellar ataxia, bipolar disorder, and peripheral neuropathy.

Another embodiment provides the peptide for use in the treatment of a monogenic hereditary disease selected from the group consisting of: Wolcott-Rallison syndrome, Wolfram syndrome, Marinesco-Sjögren syndrome, Machado-Joseph disease, and degenerative retinal diseases such as retinitis pigmentosa, and inherited nephrotic syndromes such as primary nephrotic syndrome and autosomal dominant polycystic kidney disease. Said monogenic hereditary disease is a disease having ER stress as a pathogenic component.

An embodiment provides the peptide for use according to the present disclosure, wherein said peptide is administered by peripheral administration such as intravenous, intraarterial, subcutaneous, intranasal, intraocular, intratympanic, or topical administration, enteral, parenteral or topical routes including oral, rectal, sublingual or buccal administration, intraperitoneal, intramuscular, intraarticular, transdermal, intracochlear, topic ocular, or inhalational administration, or intracranial, intrathecal, epidural or intralesional administration.

### Pharmaceutical compositions

One or more of the peptides disclosed herein can be formulated for use as or in pharmaceutical compositions. Such compositions can be formulated or adapted for administration to a subject via any route, e.g., any route approved by the appropriate authorities.

An embodiment provides a pharmaceutical composition comprising the peptide as described herein and at least one of the following: a pharmaceutically acceptable carrier, a pharmaceutically acceptable excipient, preservative, stabilizer and/or diluent.

In one embodiment, the present disclosure is further directed to a pharmaceutical composition comprising the peptide with the length of 8 - 32 amino acids comprising the sequence C-X₁-X₂-X₃-C (SEQ ID NO:17), E-X₄-C-X₁-X₂-X₃-C-A-E (SEQ ID NO:18), X₅-X₆-X₇-X₈-E-X₄-C-X₁-X₂-X₃-C-A-E-X₉-X₁₀-X₁₁ (SEQ ID NO: 19) or X₁₂-X₁₃-X₁₄-X₁₅-X₁₆-X₁₇-X₁₈-X₁₉-X₂₀-X₂₁-X₂₂-X₂₃-V-X₂₄-E-L-K-X₂₅-X₂₆-L-X₅-X₆-X₇-X₈-E-X₄-C-X₁-X₂-X₃-C-A-E-X₉-X₁₀-X₁₁ (SEQ ID NO: 20), wherein the peptide is a retro-inverso form of an amino acid sequence of any of the aforementioned sequences.

In some instances, pharmaceutical compositions can include an effective amount of one or more peptides. The terms "effective amount" and "effective to treat," as used herein, refer to an amount or a concentration of one or more compounds or a pharmaceutical composition described herein utilized for a period of time (including acute or chronic administration and periodic or continuous administration) that is effective within the context of its administration for causing an intended effect or physiological outcome.

In one embodiment of the present invention, the peptide can be incorporated into pharmaceutical compositions. Such compositions of the disclosure are prepared for storage by mixing the peptide having the desired degree of purity with optional physiologically acceptable carriers (such as nanocarriers), excipients, buffers or stabilizers (Remington's Pharmaceutical Sciences, 22nd edition, Allen, Loyd V., Jr, Ed., (2012)), in the form of lyophilized cake or aqueous solutions. Acceptable carriers, excipients, or stabilizers are non-toxic to recipients at the dosages and concentrations employed, and include buffers such as phosphate, citrate, and other organic acids; antioxidants including ascorbic acid; low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, arginine, or lysine; monosaccharides, disaccharides, and other carbohydrates including glucose, mannose, or dextrins; chelating agents such as EDTA; sugar alcohols such as mannitol or sorbitol; salt-forming counter-ions such as sodium; and/or non-ionic surfactants such as Tween, Pluronics, polyethylene glycol (PEG), or excipients that are used to enhance nose-to-brain delivery, such as chitosan, methylated pectin, alkylsaccharide-based mucosal absorption enhancers, and hydroxy fatty-acyl esters of PEG.

The actual dosage amount of the peptide (e.g., an effective amount) that is administered to a patient can be determined by physical and physiological factors such as body weight, severity of condition, the type of disease being treated, previous or concurrent therapeutic interventions, idiopathy of the patient and on the route of administration. The practitioner responsible for administration can determine the concentration of active ingredient(s) in a composition and appropriate dose(s) for the individual subject.

The peptides may also be entrapped in microcapsules prepared, for example, by coacervation techniques or by interfacial polymerization (for example, hydroxymethylcellulose or gelatin-microcapsules and poly-(methylmethacylate) microcapsules, respectively), in colloidal drug delivery systems (for example, liposomes, albumin microspheres, microemulsions, nano-particles, and nanocapsules), or in macroemulsions. Such techniques are disclosed in Remington's Pharmaceutical Sciences, 22nd edition, Allen, Loyd V., Jr, Ed., (2012).

In an embodiment, pharmaceutical compositions may comprise, for example, at least about 0.1% of an active compound. In other embodiments, an active compound may comprise between about 2% to about 75% of the weight of the unit, or between about 25% to about 60%, for example, and any range derivable therein.

In other non-limiting examples, a dose of a pharmaceutical composition or formulation can comprise from about 1 ng/kg/body weight of the peptide, about 5 ng/kg/body weight, about 10 ng/kg/body weight, about 50 ng/kg/body weight, about 100 ng/kg/body weight, about 200 ng/kg/body weight, about 350 ng/kg/body weight, about 500 ng/kg/body weight, 1 µg/kg/body weight, about 5 µg/kg/body weight, about 10 µg/kg/body weight, about 50 µg/kg/body weight, about 100 µg/kg/body weight, about 200 µg/kg/body weight, about 350 µg/kg/body weight, about 500 µg/kg/body weight, about 1 mg/kg/body weight, about 5 mg/kg/body weight, about 10 mg/kg/body weight, about 50 mg/kg/body weight, about 100 mg/kg/body weight, about 200 mg/kg/body weight, about 350 mg/kg/body weight, about 500 mg/kg/body weight, to about 1000 mg/kg/body weight of the peptide more per administration, and any range derivable therein. In non-limiting examples of a derivable range from the numbers listed herein, a range of about 5 mg/kg/body weight to about 100 mg/kg/body weight, about 5 µg/kg/body weight to about 500 mg/kg/body weight of peptide, etc., can be administered, based on the numbers described above.

The methods herein contemplate administration of an effective amount of compound or compound composition to achieve the desired or stated effect. Typically, the pharmaceutical compositions of this disclosure will be administered from about 1 to about 6 times per day or, alternatively, as a continuous infusion. Such administration can be used as a chronic or acute therapy. The amount of active ingredient that may be combined with the carrier materials to produce a single dosage form will vary depending upon the host treated and the particular mode of administration. A typical preparation will contain from about 5% to about 95% active compound (w/w). Alternatively, such preparations contain from about 20% to about 80% active compound.

Dosing can be determined using various techniques. The selected dosage level can depend upon a variety of factors, including, e.g., the activity of the particular compound employed, the route of administration, the time of administration, the rate of excretion or metabolism of the particular compound being employed, the duration of the treatment, other drugs, compounds, and/or materials used in combination with the particular compound employed, the age, sex, weight, condition, general health, and/or prior medical history of the patient being treated, and like factors well known in the medical arts. The dosage values can also vary with the severity of the condition to be alleviated. For any particular subject, specific dosage regimens can be adjusted over time according to the individual need and the professional judgment of the person administering or supervising the administration of the compositions.

In some aspects, a suitable daily dose of a compound of the disclosure can be that amount of the compound which is the lowest dose effective to produce a therapeutic effect. Such an effective dose will generally depend upon the factors described above. The precise time of administration and amount of any particular compound that will yield the most effective treatment in a given patient will depend upon the activity, pharmacokinetics, and bioavailability of a particular compound, physiological condition of the patient (including age, sex, disease type and stage, general physical condition, responsiveness to a given dosage and type of medication), route of administration, and the like.

A physician or veterinarian can prescribe the effective amount of the pharmaceutical composition required. For example, the physician or veterinarian could start doses of the compounds of the disclosure employed in the pharmaceutical composition at levels lower than that required in order to achieve the desired therapeutic effect and gradually increase the dosage until the desired effect is achieved.

Pharmaceutical compositions described herein can be in unit dosage forms suitable for single administration of precise dosages. In unit dosage form, the formulation is divided into unit doses containing appropriate quantities of one or more compounds. The unit dosage can be in the form of a package containing discrete quantities of the formulation. Non-limiting examples are liquids in vials or ampoules. Aqueous suspension compositions can be packaged in single-dose non-reclosable containers. Multiple-dose reclosable containers can be used, for example, in combination with a preservative. Formulations for parenteral injection can be presented in unit dosage form, for example, in ampoules, or in multi dose containers with a preservative.

The term "pharmaceutically-acceptable carrier or adjuvant" refers to a carrier or adjuvant that may be administered to a patient, together with a compound of this invention, and which does not destroy the pharmacological activity thereof and is nontoxic when administered in doses sufficient to deliver a therapeutic amount of the compound.

Pharmaceutically-acceptable carriers, adjuvants and vehicles that may be used in the pharmaceutical compositions of the present disclosure include, but are not limited to, ion exchangers, alumina, aluminum stearate, lecithin, self-emulsifying drug delivery systems (SEDDS) such as D-alpha-tocopherol polyethyleneglycol 1000 succinate, surfactants used in pharmaceutical dosage forms such as Tweens or other similar polymeric delivery matrices, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

The pharmaceutical compositions of the present disclosure may contain any conventional non-toxic pharmaceutically-acceptable carriers, adjuvants, or vehicles. In some cases, the pH of the formulation may be adjusted with pharmaceutically-acceptable acids, bases, or buffers to enhance the stability of the formulated compound or its delivery form. The term parenteral as used herein includes parenteral, epidural, subcutaneous, intra-cutaneous, intra-venous, intra-muscular, intra-articular, intra-arterial, intra-synovial, intra-sternal, intra-thecal, intra-lesional and intra-cranial injection or infusion techniques.

An effective amount of a compound of the disclosure can be administered in either single or multiple doses by any of the accepted modes of administration. Regardless of the route of administration selected, the compounds of the present disclosure, and/or the pharmaceutical compositions of the present disclosure, are formulated into pharmaceutically-acceptable dosage forms. The compounds according to the disclosure can be formulated for administration in any convenient way for use in human or veterinary medicine, by analogy with other pharmaceuticals.

In one aspect, the disclosure provides pharmaceutical formulation comprising a therapeutically-effective amount of one or more of the compounds described above, formulated together with one or more pharmaceutically acceptable carriers (additives) and/or diluents. In one aspect, one or more of the compounds described herein are formulated for parenteral administration for parenteral administration, one or more compounds disclosed herein can be formulated as aqueous or non-aqueous solutions, dispersions, suspensions, or emulsions or sterile powders which can be reconstituted into sterile injectable solutions or dispersions just prior to use. Such formulations can comprise sugars, alcohols, antioxidants, buffers, bacteriostats, solutes which render the formulation isotonic with the blood of the intended recipient or suspending or thickening agents. These compositions can also contain adjuvants such as preservatives, wetting agents, emulsifying agents and dispersing agents. Prevention of the action of microorganisms upon the subject compounds can be ensured by the inclusion of various antibacterial and antifungal agents, for example, paraben, chlorobutanol, phenol sorbic acid, and the like. It can also be desirable to include isotonic agents, such as sugars, sodium chloride, and the like into the compositions. In addition, prolonged absorption of the injectable pharmaceutical form can be brought about by the inclusion of agents which delay absorption such as aluminum monostearate and gelatin. If desired, the formulation can be diluted prior to use with, e.g., an isotonic saline solution or a dextrose solution. In some examples, the compound is formulated as an aqueous solution and is administered intravenously.

Pharmaceutical compositions can be in the form of a solution or powder for injection. Such compositions may be formulated according to techniques known in the art using suitable dispersing or wetting agents (such as, for example, Tween 80) and suspending agents. The sterile injectable preparation may also be a sterile injectable solution or suspension in a non-toxic parenterally acceptable diluent or solvent, for example, as a solution in 1,3-butanediol. Among the acceptable vehicles and solvents that may be employed are mannitol, water, Ringer's solution, and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, or carboxymethyl cellulose or similar dispersing agents which are commonly used in the formulation of pharmaceutically-acceptable dosage forms such as emulsions and or suspensions. Other commonly used surfactants such as Tweens or Spans and/or other similar emulsifying agents or bioavailability enhancers which are commonly used in the manufacture of pharmaceutically-acceptable solid, liquid, or other dosage forms may also be used for the purposes of formulation.

Pharmaceutical compositions can be orally administered in any orally acceptable dosage form including, but not limited to, capsules, tablets, emulsions and aqueous suspensions, dispersions and solutions. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions and/or emulsions are administered orally, the active ingredient may be suspended or dissolved in an oily phase is combined with emulsifying and/or suspending agents. If desired, certain sweetening and/or flavoring and/or coloring agents may be added.

The pharmaceutical compositions of the present disclosure may also be administered in the form of suppositories for rectal administration. These compositions can be prepared by mixing a compound of the present disclosure with a suitable non-irritating excipient that is solid at room temperature but liquid at the rectal temperature and therefore will melt in the rectum to release the active components. Such materials include, but are not limited to, cocoa butter, beeswax and polyethylene glycols.

Alternatively or in addition, pharmaceutical compositions can be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art.

In some instances, one or more peptides disclosed herein can be conjugated, for example, to a carrier protein. Such conjugated compositions can be monovalent or multivalent. For example, conjugated compositions can include one peptide disclosed herein conjugated to a carrier protein. Alternatively, conjugated compositions can include two or more peptides disclosed herein conjugated to a carrier.

Provided herein are methods of using a peptide described herein. For example, the methods provided herein can include administering a peptide as described herein to a patient. A patient can include both mammals and non-mammals.

A pharmaceutically acceptable carrier can be selected on the basis of the selected route of administration and standard pharmaceutical practice. For example, the compositions can be formulated into suitable pharmaceutical preparations such as solutions, suspensions, tablets, dispersible tablets, pills, capsules, powders, sustained release formulations or elixirs, for oral administration or in sterile solutions or suspensions for parenteral administration and intraperitoneal injection, as well as transdermal patch preparation, dry powder inhalers, and ointments (see, e.g., Ansel, Introduction to Pharmaceutical Dosage Forms, Fourth Edition 1985, 126). A peptide and/or an immunoglobulin may be formulated into dosage forms according to standard practices in the field of pharmaceutical preparations. See Alphonso Gennaro, ed., Remington's Pharmaceutical Sciences, 18th Edition (1990), Mack Publishing Co., Easton, Pa.

For parenteral administration, a pharmaceutical composition can include a suitable carrier or diluent such as water, an oil (particularly a vegetable oil), ethanol, saline solution, aqueous dextrose (glucose) and related sugar solutions, glycerol, or a glycol such as propylene glycol or polyethylene glycol. Solutions for parenteral administration preferably contain a water soluble salt of a peptide and/or an active agent. Stabilizing agents, antioxidant agents and preservatives may also be added. Suitable antioxidant agents include sulfite, ascorbic acid, citric acid and its salts, and sodium EDTA. Suitable preservatives include benzalkonium chloride, methyl- or propyl-paraben, and chlorbutanol. The composition for parenteral administration may take the form of an aqueous or non-aqueous solution, dispersion, suspension or emulsion.

For oral administration, a pharmaceutical composition can include one or more solid inactive ingredients for the preparation of tablets, capsules, pills, powders, granules or other suitable oral dosage forms. For example, a pharmaceutical composition can include at least one excipient such as fillers, binders, humectants, disintegrating agents, solution retarders, absorption accelerators, wetting agents absorbents or lubricating agents.

The disclosure also features a pharmaceutical composition that can further include a neural cell. The neural cell can be, for example, a neuron, a neural stem cell, or a neuronal precursor cell.

The present disclosure relates to the pharmaceutical composition comprising the peptide as described herein and at least one of the following: a pharmaceutically acceptable carrier, excipient, preservative, stabilizer and/or diluent for use as a medicament.

In a method of treatment, a pharmaceutically effective amount of the peptide as defined herein is administered to a patient. In other words, the peptide according to the present disclosure is for use in the treatment of a degenerative, chronic, or progressive disease or disorder, such as a CNS disease or disorder, a monogenic hereditary disease (having ER stress as a pathogenic component).

The pharmaceutical composition is for use in the treatment of a degenerative, chronic, or progressive disease or disorder, such as a neurodegenerative disease or disorder.

Said neurodegenerative disease or disorder is a central nervous system disease selected from the group consisting of: Parkinson's disease, Alzheimer's disease, multiple system atrophy, amyotrophic lateral sclerosis, frontotemporal lobar degeneration, dementia with Lewy bodies, mild cognitive impairment, Huntington's disease, traumatic brain injury, traumatic spinal cord injury, progressive supranuclear palsy, Pick's disease, pure autonomic failure, corticobasal degeneration, chronic traumatic encephalopathy, spinocerebellar ataxia, and peripheral neuropathy.

The route of peptide administration is in accord with known methods as well as the general routes of injection or infusion by intravenous, intraarterial, subcutaneous, intranasal, intraocular, intratympanic, or topical administration, enteral, parenteral or topical routes including oral, rectal, sublingual or buccal administration, intracranial, intrathecal or epidural, intraperitoneal, intramuscular, intraarticular, transdermal, intracochlear, topic ocular, intralesional, or inhalational administration, or sustained release systems as noted below.

Suitable examples of sustained-release preparations include semipermeable matrices of solid hydrophobic polymers containing the peptide, which matrices are in the form of shaped articles, e.g., films, or microcapsules. Examples of sustained-release matrices include polyesters, hydrogels as described by Langer et al., J. Biomed. Mater. Res., 15: 167-277 (1981) and Langer, Chem. Tech., 12:98-105 (1982) or polyvinylalcohol, polylactides (U.S. Pat. No. 3,773,919, EP 58,481), or non-degradable ethylene-vinyl acetate (Langer et al., supra).

The present disclosure is also directed to methods for treatment of a degenerative, chronic, or progressive disease or disorder, such as a CNS disease or disorder, a monogenic hereditary disease (having ER stress as a pathogenic component), wherein a pharmaceutically effective amount of the peptide as defined herein is administered to a patient. Preferably, said fragment is administered peripherally. Oral administration is also a preferred form of administration.

The present disclosure is also directed to a use of the peptide as defined herein for the manufacture of a medicament for the treatment of a degenerative, chronic, or progressive disease or disorder, such as a CNS disease or disorder, or a monogenic hereditary disease (having ER stress as a pathogenic component).

The present disclosure relates to a method for treating a degenerative, chronic, or progressive disease or disorder, such as a neurodegenerative disease or disorder in a subject in need thereof, the method comprising administering to the subject a pharmaceutical composition comprising a peptide as described herein.

In an embodiment, a method for treating a neurodegenerative disease or disorder such as a central nervous system disease selected from the group consisting of: Parkinson's disease, Alzheimer's disease, multiple system atrophy, amyotrophic lateral sclerosis, frontotemporal lobar degeneration, dementia with Lewy bodies, mild cognitive impairment, Huntington's disease, traumatic brain injury, traumatic spinal cord injury, progressive supranuclear palsy, Pick's disease, pure autonomic failure, corticobasal degeneration, chronic traumatic encephalopathy, spinocerebellar ataxia, and peripheral neuropathy, comprises administering to the subject a pharmaceutical composition comprising a peptide as described herein.

The present disclosure relates to a method for treating a monogenic hereditary disease selected from the group consisting of: Wolcott-Rallison syndrome, Wolfram syndrome, Marinesco-Sjögren syndrome, Machado-Joseph disease, and degenerative retinal diseases such as retinitis pigmentosa, and inherited nephrotic syndromes such as primary nephrotic syndrome and autosomal dominant polycystic kidney disease, the method comprising administering to the subject a pharmaceutical composition comprising a peptide as described herein. Said monogenic hereditary disease has ER stress as a pathogenic component.

The subject in need may be human.

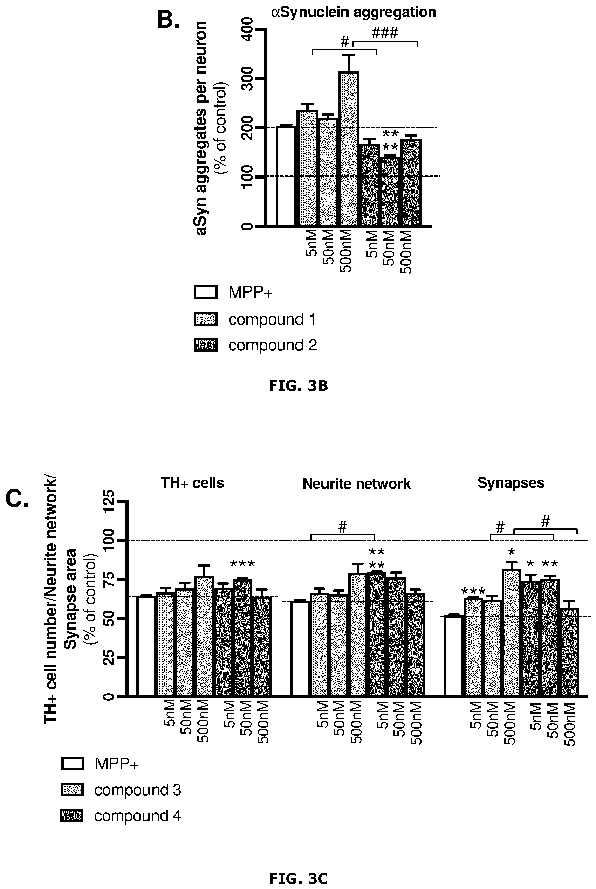

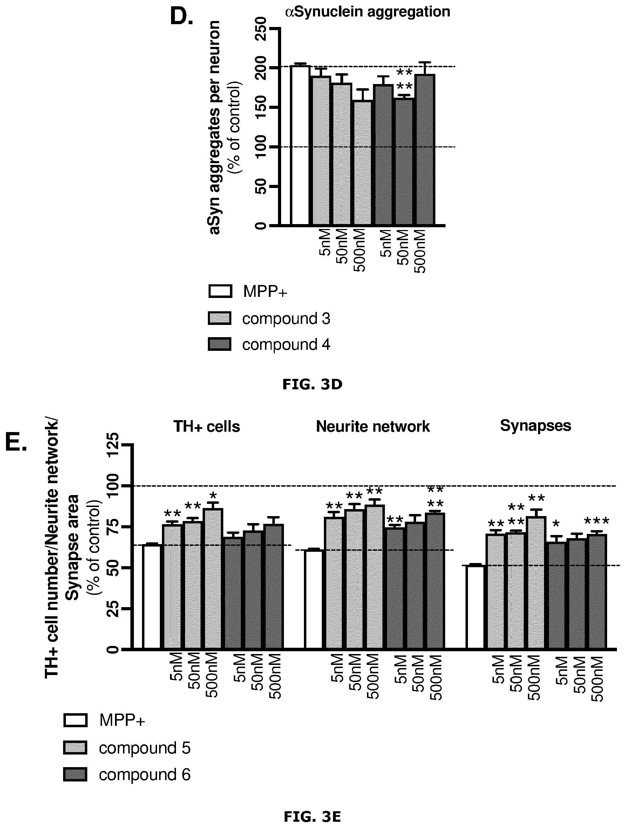

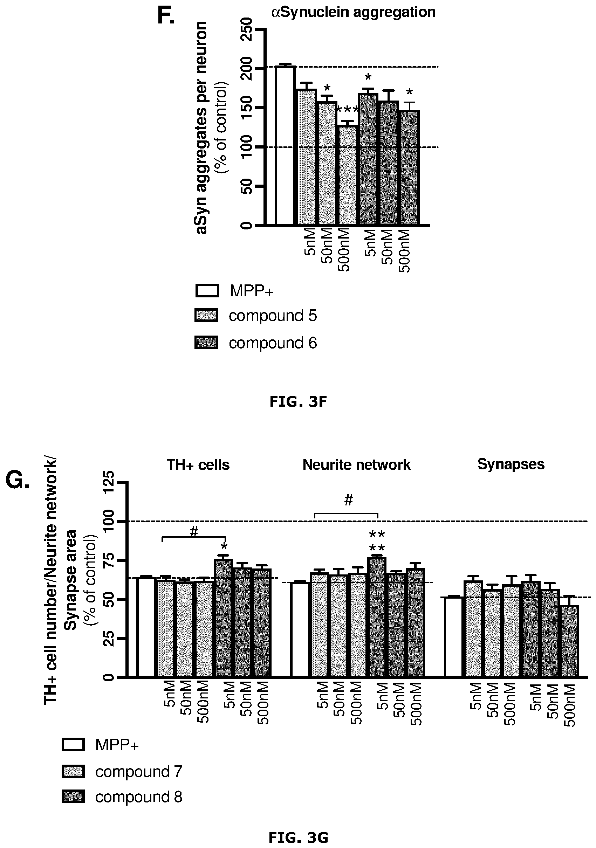

The peptide of the present disclosure or a pharmaceutical composition comprising said peptide can be administered continuously by infusion or by bolus injection. Generally, where the disorder permits, one should formulate and dose the fragment for site-specific delivery. Administration can be continuous or periodic. Administration can be accomplished by a constant- or programmable-flow implantable pump or by periodic injections. Peripheral or systemic administration is preferred as the present disclosure shows that retro-inverso peptides are capable of effective penetration through the neuronal cell membrane and through *in vitro* blood-brain-barrier (Figures 3, 4 and 8). Other preferred administration routes are subcutaneous, intrathecal, intracerebroventricular, intranasal, or transdermal administration.

In another embodiment, the present disclosure provides a method for promoting survival of dopaminergic neurons comprising the step of contacting dopaminergic neurons with the peptide of 8 - 32 amino acids comprising the sequence SEQ ID NO: 17, SEQ ID NO: 18, SEQ ID NO: 19 or SEQ ID NO:20, wherein the peptide comprises a retro-inverso form of said amino acid sequence. Preferably, the method is performed *in vitro* as shown below in the Experimental Section. Said dopaminergic neurons are preferably cultured non-human neurons, such as mouse or rat sympathetic neurons, or human neurons derived from induced pluripotent cells (iPSC).

Based on the results provided by the present disclosure, the disclosure is also directed to a peptide with the length of 8 - 32 amino acids comprising the sequence SEQ ID NO: 17, SEQ ID NO: 18, SEQ ID NO: 19 or SEQ ID NO:20, wherein the peptide comprises a retro-inverso form of said amino acid sequence, for use in the treatment of a degenerative, chronic, or progressive disease or disorder, such as a CNS disease or disorder, or disorder, a monogenic hereditary disease (having ER stress as a pathogenic component).

### Methods of making the peptides

Methods of synthesizing the compounds of the present disclosure are known in the art. The following exemplary method may be used. It will be appreciated that the various steps may be performed in an alternate sequence or order to give the desired compounds. Synthetic chemistry transformations and protecting group methodologies (protection and deprotection) useful in synthesizing the compounds described herein are known in the art and include, e.g., those such as described in R. Larock, Comprehensive Organic Transformations, VCH Publishers (1989); T. W. Greene and P. G. M. Wuts, Protective Groups in Organic Synthesis, 3d. Ed., John Wiley and Sons (1999); L. Fieser and M. Fieser, Fieser and Fieser's Reagents for Organic Synthesis, John Wiley and Sons (1994); and L. Paquette, ed., Encyclopedia of Reagents for Organic Synthesis, John Wiley and Sons (1995), and subsequent editions thereof.

The peptides of the present disclosure can be made by chemical synthesis methods, which are well known to the ordinarily skilled artisan. See, e.g., Fields et al., Chapter 3 in Synthetic Peptides: A User's Guide, ed. Grant, W. H. Freeman & Co., New York, N.Y., 1992, p. 77.

One manner of making of the peptides described herein is using solid phase peptide synthesis (SPPS). The C-terminal amino acid is attached to a cross-linked polystyrene resin via an acid labile bond with a linker molecule. This resin is insoluble in the solvents used for synthesis, making it relatively simple and fast to wash away excess reagents and by-products. The N-terminus is protected with the Fmoc group, which is stable in acid, but removable by base. Any side chain functional groups are protected with base stable, acid labile groups.

The publications and other materials used herein to illuminate the background of the invention, and in particular, to provide additional details with respect to its practice, are incorporated herein by reference.

It is apparent to a person skilled in the art that as technology advanced, the basic idea of the disclosure can be implemented in various ways. The disclosure and its embodiments are therefore not restricted to the below examples, but they may vary within the scope of the claims.

### EXAMPLES

### Example 1

### Neuroprotective effect of parent and retro-inverso compounds on dopaminergic TH-positive neurons injured with MPP+

Neuroprotective effects of compounds were tested in an *in vitro* model in which primary cultures of rat embryonic mesencephalic neurons were stressed with MPP+, the active metabolite of neurotoxin 1-methyl-4-phenyl-1,2,3,6-tetrahydropyridine (MPTP). MPP+ kills dopaminergic (TH-positive) neurons via a variety of toxic mechanisms, including mitochondrial dysfunction, generation of peroxynitrite, oxidative stress, ER stress and induction of apoptosis. Since peptides derived from the C-terminal domain of CDNF and MANF were modified into retro-inverso peptides, their neuroprotective activity was tested in a model where full-length CDNF protein has shown to be neuroprotective (Figs. 2A and 2B).

### Materials and Methods

### Materials and methods of synthesis and characterization of peptides.

Standard solid phase peptide synthesis methods were used for production of linear (L-aa) and retro-inverso (D-aa) peptides.

### General protocol used to synthesise the peptides - SPPS

Solid phase peptide synthesis was carried out at Alstra Biotage Initiator peptide synthesizer. Standard Fmoc protected amino acids were used for peptide elongation: Ala, Arg(Pbf), Asp(tBu), Gln(Trt), Glu(OtBu), Gly, His(Trt), Ile, Lys(Boc), Leu, Met, Ser(tBu), Thr(tBu), Trp(Boc), Val and Cys(StBu). Removal of Fmoc group was performed using 20% piperidine in DMF, coupling was performed using 4 eq of corresponding amino acid, 3.9 eq of HBTU, 4 eq of HOBt, and 8 eq of DIPEA under microwave irradiation. The crude peptides were deprotected and cleaved from the resin through a treatment with TFA/H₂O/iPr₃SiH for 2h followed by precipitation in cold Et₂O.

In case of the Retro inverso peptides, the standard Fmoc protected D-amino acids acids were used instead of Fmoc protected L-amino acids. The reversed sequence was taken care of before initiating the Biofage Initiator peptide synthesizer, so that the required retro-inverso peptide is obtained.

The quality control of the peptides was obtained using Standard liquid chromatography-mass spectrometry methods. Figure 1 (column 5) shows the peaks identified in MS analysis of the peptides

### Culture of mesencephalic neurons.

Rat dopaminergic neurons were cultured as described by Visanji et al., 2008. Briefly, the midbrains obtained from 15-day-old rat embryos (Janvier, France) were dissected, and the ventral portion of the mesencephalic flexure, a region of the developing brain rich in dopaminergic neurons, was used for the cell preparations. The midbrain cells were dissociated by trypsinization for 20 min at 37°C (solution at a final concentration of 0.05% trypsin and 0.02% EDTA). The reaction was stopped by adding Dulbecco's modified Eagle's medium (DMEM) containing DNAase I grade II (0.5 mg/mL) and 10% of fetal calf serum (FCS). Cells were then mechanically dissociated by 3 passages through a 10 ml pipette. Cells were then centrifuged at 180 x g for 10 min at +4°C on a layer of BSA (3.5%) in L15 medium. The cell pellets was re-suspended in a defined culture serum-free medium consisting of Neurobasal (Invitrogen) supplemented with B27 (2%), L-glutamine (2 mM) and 2% of PS solution and 10 ng/ml of Brain-derived neurotrophic factor (BDNF) and 1 ng/mL of Glial-Derived Neurotrophic Factor (GDNF). Viable cells were counted in a Neubauer cytometer using the trypan blue exclusion test. The cells were seeded at a density of 40 000 cells/well in 96 well-plates (pre-coated with poly-L-lysine) and maintained in a humidified incubator at 37°C in 5% CO₂/95% air atmosphere. Half of the medium was changed every 2 days with fresh medium. On 96-wells plates, only 60 wells are used. To avoid any edge effect, the first and last lines and columns were not be used for culture and were filled with sterile water.

### Test compounds and MPP+ exposure.

On day 6 of culture, CDNF or the compounds 1-16 (SEQ ID NO:s 1 - 16, parent and retro-inverso), were dissolved in culture medium and then pre-incubated with mesencephalic neurons for 4 hours before the MPP+ application. Four hours after the preincubation of the compounds, MPP+ was added to a final concentration of 4 µM, diluted in control medium still in presence of compounds for 48 h.

### Immunostaining: TH neuron survival neurite network, and α-syn aggregation in TH neurons.

48 hours after intoxication, the cells were fixed by a solution of 4% paraformaldehyde in PBS, pH 7.3 for 20 min at room temperature. The cells were washed twice in PBS, and then permeabilized and non-specific sites were blocked with a solution of PBS containing 0.1% of saponin and 1% FCS for 15 min at room temperature. Next, the cells were incubated with (a) a monoclonal antibody anti-Tyrosine Hydroxylase (TH) produced in mouse at dilution of 1:10000 and with (b) a polyclonal antibody anti-alpha synuclein (aSyn) antibody produced in rabbit at dilution of 1:400 in PBS containing 1% FCS, 0.1 % saponin, for 2 h at room temperature. These antibodies were revealed with a secondary antibody Alexa Fluor 488 goat anti-mouse IgG at the dilution 1:800 and with an Alexa 568 goat anti rabbit IgG at the dilution 1:400 in PBS containing 1% FCS, 0.1 % saponin, for 1 h at room temperature.

### Synapse immunostaining: TH neuron and PSD-95 (overlap between TH/PSD-95 neurons).

48 hours after intoxication, the cell culture supernatant was removed, and the cells fixed by a solution of 4% paraformaldehyde in PBS, pH 7.3 for 20 min at room temperature. The cells were washed twice in PBS, and then permeabilized and non-specific sites were blocked with a solution of PBS containing 0.1% of saponin and 1% FCS for 15 min at room temperature. Then, the cells were incubated with a) a monoclonal Anti-Tyrosine Hydroxylase (TH) antibody produced in mouse at dilution of 1:10000 in PBS containing 1% FCS, 0.1 % saponin, for 2 hours at room temperature, and b) a polyclonal anti-postsynaptic density protein-95 (PSD-95) antibody produced in rabbit at dilution of 1:200 in PBS containing 1% FCS, 0.1 % saponin, for 2 h at room temperature. This antibody stains specifically synapses. These antibodies were revealed with Alexa Fluor 488 goat anti-mouse IgG at the dilution 1:800 and with Alexa Fluor 568 goat anti-rabbit IgG at the dilution 1:400 in PBS containing 1% FCS, 0.1 % saponin, for 1 h at room temperature.

For each condition, pictures representing the whole well area were automatically acquired using ImageXpress (Molecular device) at 10x (20 pictures, for TH and a-syn) or at 40x magnification (60 pictures, for TH and PSD-95). The following read-out were automatically determined by using Custom Module Editor (Molecular Devices):
- Analysis of total number of TH neurons (TH positive neurons),
- total neurite network of TH positive neurons (in µm)
- aSyn aggregation (overlapping between TH and aSyn staining)
- number of synapses TH positive neurons (overlap of TH and PSD95 in µm²).

Total number of TH+ neurons, total neurite network of TH+ neurons and the number of synapses of TH+ neurons are presented in Figure 2A (CDNF), Figure 3A (Compound 1 and 2), Figure 3C (Compound 3 and 4), Figure 3E (Compound 5 and 6), Figure 3G (Compound 7 and 8), Figure 4A (Compound 9 and 10), and Figure 4C (Compound 11 and 12), Figure 4E (Compounds 13 and 14), Figure 4G (Compounds 15 and 16).

α-synuclein aggregation in TH-positive neurons of a primary culture of mesencephalic cells after MPP+ injury is presented in Figure 2B (CDNF), Figure 3B (Compound 1 and 2), 3D (Compound 3 and 4), Figure 3F (Compound 5 and 6), Figure 3H (Compound 7 and 8), Figure 4B (Compound 9 and 10), and Figure 4D (Compound 11 and 12), Figure 4F (Compounds 13 and 14), Figure 4H (Compounds 15 and 16).

The data shows that many retro-inverso peptides protected TH-positive neurons and their neurites and synapses from MPP+ toxicity. Moreover, these retro-inverso compounds effectively reduced the number of aSyn inclusions, whose accumulation is strongly induced by MPP+, in TH-positive neurons. In most cases the potency of retro-inverso peptides (compounds 2, 4, 6, 8, 10, 12, 14 and 16) was comparable to their parent compounds (compounds 1, 3, 5, 7, 9, 11, 13 and 15, respectively) and to full-length CDNF protein (Fig. 2).

### Example 2

### GRP78 binding of the compounds

CDNF and MANF protect cells from ER stress-induced apoptosis by modulating cellular responses to ER stress. Yan et al (2019) showed that the C-terminus of MANF binds to the nucleotide-binding domain (NBD) of GRP78 and regulates its cellular activities. This data suggests that MANF (and CDNF) have a regulatory interaction rather substrate-like interaction with GRP78, the most abundant chaperone protein in the ER lumen. As GRP78 also serves as a critical ligand of the three receptors of the unfolded protein response (UPR) pathway, IRE1α, PERK and ATF6, it seems that the GRP78 interaction may be a central protein-protein interaction of CDNF and MANF that could mediate their cytoprotective effects. Therefore, we tested binding of CDNF and MANF-derived compounds for their GRP78 binding affinity.

### Materials and methods

### Molecular modeling.

The GRP78-NBD in complex with different compounds mentioned here (Fig 2A-2P) were modelled on the basis of the previously solved structure of the GRP78-NBD:MANF complex using the PRIME module of Schrodinger suite version 2018-4 (Schrödinger Llc, USA) via the MAESTRO interface. The model generated was manually checked using the template structure (PDB: 6HAB, Yan et al, 2019). The model was also verified looking into its Ramachandran diagram.

### Cell-free binding assay.

His tagged active GRP78 was purchased from StressMarq (#PR484426) and was labelled using Red-Tris-NTA dye (10-fold excess of protein to dye ratio; Nanotemper Technologies GmbH). Binding of different peptides (in serial dilution) to labelled GRP78 was measured in a Monolith N.T standard capillary using a Monolith N.A. device (Nanotemper Technologies GmbH) at high power in a PBS environment in the presence of 0.5% Tween-20. Fig. 5B shows tabulated data obtained from MST binding experiments.

### Example 3

### In vitro metabolic stability of parent and retro-inverso compounds in rat plasma

Metabolic stability was studied using rat plasma over the time period of 120 min, with initial test concentration of 1 µM. Samples were analysed using LC/QE-orbitrap-MS. Calculated half-life is based on compound disappearance in rat plasma.

### Materials and Methods

The parent or retro-inverso compounds 1 - 8 (SEQ ID NO:s 1 - 8) were incubated in concentration 1 µM with rat plasma (Spraque-Dawley, male; 400 µl) for different time points (0, 20, 40, 60 or 120 min) in 37°C. The incubation was terminated by acetonitrile. The collected samples were centrifuged for 20 min at 2272 × g and analyzed. Stock solutions was prepared using 50% DMSO, and the compounds were spiked 1/100 to incubation to have final DMSO content of 0.5%. The samples were analyzed by UHPLC/PDA with high resolution mass spectrometry (QE-Orbitrap-MS on DDI mode) to monitor disappearance of the compound. Enalapril 1 µM was used as a disappearance rate control. The analytical method was optimized by using the parent compounds for optimum chromatographic properties (peak shape and retention) and mass spectrometric ionization. The Ion chromatograms were extracted from the total ion chromatograms using calculated monoisotopic accurate masses with 5 mDa window. Disappearance was based on LC/MS peak areas, marking 0 min as 100%. The first-order rate constants k (min-1) of the metabolism were obtained from the slope of time versus logarithm (% of remaining compound) plot using Excel software. The *in vitro* half-life (t1/2) of study compound is defined as: t1/2 =ln2/k.

In Figure 6A, each pair of bars shows data for an unmodified peptide (parent) and the corresponding modified peptide (retro-inverso).

### Example 4

### In vitro metabolic stability of parent and retro-inverso compounds in human plasma

Metabolic stability was studied using human plasma over the time period of 120 min, with initial test concentration of 1 µM. Samples were analysed using LC/QE-orbitrap-MS. Calculated half-life is based on compound disappearance in human plasma.

### Materials and Methods

The parent or retro-inversed compounds 1 - 8 (SEQ ID NO:s 1 - 8) were incubated in concentration 1 µM with human plasma (mixed gender, 400 µl) for different time points (0, 20, 40, 60 or 120 min) in 37°C. The incubation was terminated by acetonitrile. The collected samples were centrifuged for 20 min at 2272 × g and analyzed. The samples were analyzed by UHPLC/PDA with high resolution mass spectrometry (QE-Orbitrap-MS on DDI mode) to monitor disappearance of the compound. Propanthelin bromide 1uM was used as a disappearance rate control. The analytical method was optimized by using the parent compounds for optimum chromatographic properties (peak shape and retention) and mass spectrometric ionization. The Ion chromatograms were extracted from the total ion chromatograms using calculated monoisotopic accurate masses with 5 mDa window. Disappearance was based on LC/MS peak areas, marking 0 min as 100%. The first-order rate constants k (min-1) of the metabolism were obtained from the slope of time versus logarithm (% of remaining compound) plot using Excel software. The *in vitro* half-life (t1/2) of study compound is defined as: t1/2 =ln2/k.

In Figure 6B, each pair of bars shows data for an unmodified peptide (parent) and the corresponding modified peptide (retro-inverso).

Peptide stability in human plasma was better for both linear and retro-inverso peptides compared to their stability in rat plasma, as shown by maximal reported half-lives in human plasma (a test-specific maximum for half-life was 795 min). One tested retro-inverso compound (compound 8) showed improved stability in rat and human plasma as compared to its parent compound (compound 7).

### Example 5

### In vitro metabolic stability of parent and retro-inversed compounds in rat hepatocytes

Metabolic stability was studied using rat (Spraque-Dawley, male) liver hepatocytes for 0 - 60 minutes (n=2) with initial test concentration of 1 µM. Samples were analysed using LC/QE-orbitrap-MS. Calculated half-life is based on compound disappearance.

### Materials and Methods

The parent or retro-inversed compounds 1 - 8 (SEQ ID NO:s 1 - 8) were incubated in concentration 1 µM with pooled cryopreserved rat hepatocytes (Spraque-Dawley, male; 400 µl, 1.0 million viable cells /ml) for different time points (0, 10, 20, 40 or 60 min) in 37°C. The cell density and viability were determined by trypan blue exclusion method. The incubation was terminated by acetonitrile. The collected samples were centrifuged for 20 min at 2272 × g and analyzed. The samples were analyzed by UHPLC/PDA with high resolution mass spectrometry (QE-Orbitrap-MS on DDI mode) to monitor disappearance of the compound. Verapramil 1uM was used as a disappearance rate control. The analytical method was optimised by using the parent compounds for optimum chromatographic properties (peak shape and retention) and mass spectrometric ionisation. The Ion chromatograms were extracted from the total ion chromatograms using calculated monoisotopic accurate masses with 5 mDa window. Disappearance was based on LC/MS peak areas, marking 0 min as 100%. The first-order rate constants k (min-1) of the metabolism were obtained from the slope of time versus logarithm (% of remaining compound) plot using Excel software. The *in vitro* half-life (t1/2) of study compound is defined as: t1/2 =ln2/k.

In Figure 7A, each pair of bars shows data for an unmodified peptide (parent) and the corresponding modified peptide (retro-inverso).

All tested retro-inverso peptides (compounds 2, 4, 6 and 8) showed significantly improved stability in rat hepatocytes as compared to their parent compounds (compounds 1, 3, 5 and 7, respectively).

### Example 6

### In vitro metabolic stability of parent and retro-inverso compounds in human hepatocytes

Metabolic stability was studied using rat (mixed gender, male) liver hepatocytes for 0 - 60 minutes (n=2) with initial test concentration of 1 µM. Samples were analysed using LC/QE-orbitrap-MS. Calculated half-life is based on compound disappearance.

### Materials and Methods

The parent or retro-inversed compounds 1 - 8 (SEQ ID NO:s 1 - 8) were incubated in concentration 1 µM with pooled cryopreserved human hepatocytes (mixed gender; 400 µl, 1.0 million viable cells /ml) for different time points (0, 10, 20, 40 or 60 min) in 37°C. The cell density and viability were determined by trypan blue exclusion method. The incubation was terminated by acetonitrile. The collected samples were centrifuged for 20 min at 2272 × g and analyzed. The samples were analyzed by UHPLC/PDA with high resolution mass spectrometry (QE-Orbitrap-MS on DDI mode) to monitor disappearance of the compound. Verapramil 1uM was used as a disappearance rate control. The analytical method was optimised by using the parent compounds for optimum chromatographic properties (peak shape and retention) and mass spectrometric ionisation. The Ion chromatograms were extracted from the total ion chromatograms using calculated monoisotopic accurate masses with 5 mDa window. Disappearance was based on LC/MS peak areas, marking 0 min as 100%. The first-order rate constants k (min-1) of the metabolism were obtained from the slope of time versus logarithm (% of remaining compound) plot using Excel software. The *in vitro* half-life (t1/2) of study compound is defined as: t1/2 =ln2/k.

In Figure 7B, each pair of bars shows data for an unmodified peptide (parent) and the corresponding modified peptide (retro-inverso).

All tested retro-inverso peptides (compounds 2, 4, 6 and 8) showed significantly improved stability in human hepatocytes as compared to their parent compounds (compounds 1, 3, 5 and 7, respectively).

### Example 7

### Penetration of parent and retro-inverso compounds through a 3D in vitro model of blood brain barrier

Due to the interest of developing CDNF- and MANF-derived peptides for treatment of CNS diseases with peripheral route of administration, the ability of the compounds to pass the blood-brain barrier was tested in an established *in vitro* model of the blood-brain barrier. For this purpose, retro-inverso and linear compounds were incubated at 500 nM for 2 h in a two-compartment *in vitro* blood-brain barrier model (n=4) followed by sample collection and LC-MS/MS analysis.

### Materials and Methods

### Primary culture of astrocytes.

Rat astrocytes were prepared from a E15 embryos. Briefly, pregnant female rats (Wistar, Janvier Labs) of 15 days of gestation were deeply anesthetized in a (CO₂ chamber) and then killed by cervical dislocation. Fetuses were collected and immediately placed in ice-cold L15 Leibovitz medium with a 2% penicillin (10,000 U/mL) and streptomycin (10 mg/mL) solution (PS) and 1% bovine serum albumin (BSA). Full brains were treated for 20 min at 37°C with a trypsin- EDTA solution at a final concentration of 0.05% trypsin and 0.02% EDTA. Dissociated cells were cultured in DMEM 10% fetal calf serum. Purified astrocytes were used at passage 4 (P4).

*Culture of human endothelial cells.* A vial of HBMEC (Primary Human Brain Microvascular Endothelial Cells, ACBRI 376) was used at a specific passage 8 (P8).
*Primary culture of cortical neurons.* Rat cortical neurons were cultured as described by Callizot et al., 2013 with modification. Briefly, pregnant female rats (Wistar, Janvier Labs) of 15 days of gestation were deeply anesthetized in a CO₂ chamber and then killed by cervical dislocation. Fetuses were collected and immediately placed in ice-cold L15 Leibovitz medium with a 2% penicillin (10,000 U/mL) and streptomycin (10 mg/mL) solution (PS) and 1% bovine serum albumin (BSA). Cortices were treated for 20 min at 37°C with a trypsin-EDTA solution at a final concentration of 0.05% trypsin and 0.02 % EDTA. The dissociation was stopped by addition of Dulbecco's modified Eagle's medium (DMEM) with 4.5 g/L of glucose, containing DNAse I grade II (final concentration 0.5 mg/mL) and 10% fetal calf serum (FCS). Cells were mechanically dissociated by three forced passages through the tip of a 10-ml pipette. Cells were then centrifuged at 515 x g for 10 min at 4°C. The pellet resuspended in a defined culture medium consisting of Neurobasal medium with a 2% solution of B27 supplement, 2 mmol/L of L-glutamine, 2% of PS solution, 10 ng/mL of brain-derived neurotrophic factor (BDNF). Viable cells were counted in a Neubauer cytometer, using the trypan blue exclusion test. The cortical neurons were seeded in the bottom of well pre-coated with poly-L-lysine at a density of 255,000 per well in 24-well plate with insert and cultured at 37°C in an air (95 %)-CO₂ (5 %) incubator. The culture medium was changed every other day.
*Co-Culture of endothelial cells, astrocytes and primary cortical neurons.* The procedure was performed as previously published (Xue et al., 2013 with modifications, Callizot et al., 2017). Briefly, on day 0, purified astrocytes (P4) were rapidly thawed in a water bath at 37°C. The cells were immediately put in DMEM containing 10% of FCS. Cell suspension was centrifuged at 515 x g for 5 min at 4°C and the pellets were suspended in DMEM F12 containing 10 % of FCS. Cells were seeded in the outer side of the insert membrane (PET, 1 µm) at the density of 45,000 cells per insert and cultured at 37°C in an air (95 %)-CO2 (5 %) incubator. Thirty-six (36) hours after the astrocytes seeding, HBMEC (P8) were rapidly thawed in a water bath at 37°C and immediately put in DMEM containing 10% of FCS. Cell suspension was centrifuged at 515 x g for 5 min at 4 °C and the pellets were suspended in EGM-2 bullet kit containing 5 % of FCS, 1% of PS solution, 1.4 µM of Hydrocortisone, 5 µg/mL of acid ascorbic, 1 % of lipid mixture, 10 mM of HEPES, 1ng/mL of bFGF. Cells were seeded in the inner side of the insert membrane (PET, 1µm) at the density of 50,000 cells per insert and were cultured at 37 °C in an air (95 %)-CO₂ (5 %) incubator. Thirty-six (36) hours after the HBMEC seeding (72 hours after the seeding of astrocytes), the cortical neurons were seeded in the poly-L-lysine pre-coated well-bottom at a density of 170,000 per well and were cultured at 37°C in an air (95 %)-CO₂ (5 %) incubator.

*Parent or retro-inverso compounds application.* Five (5) days after HBMEC seeding, following the first testing of the integrity of endothelial cell layer, the test compounds 1 - 8 (SEQ ID NO:s 1 - 8) were added to the luminal compartment and incubated for 2 hours in concentration 500 nM.
*Quantification of test compounds.* The detection and quantification of each compound in abluminal supernatants were further performed by mass-spectrometry (MS) analysis. After sample thawing, a 100 µL aliquot of each cell culture sample was analysed by quantifying the peptides by mass spectroscopy.
The percentage of passage calculated represents the percentage of compound applied in the abluminal compartment that was measured in the abluminal compartment at the end of the application.
In Figure 8 each pair of bars shows data for an unmodified peptide (parent) and the corresponding modified peptide (retro-inverso). Better BBB passage was observed for retro-inverso compounds 2, 4, and 6 (SEQ ID NO:s 2, 4, 6) compared with the parent compounds.
Three tested retro-inverso compounds (compounds 2, 4 and 6) showed improved ability to pass through the *in vitro* blood-brain barrier as compared to their parent compounds (compounds 1, 3 and 5, respectively).

### Example 8

### In vivo pharmacokinetic of retro-inverso compounds after peripheral administration to rats

The clearance and elimination of peptides can be mediated by multiple processes *in vivo,* including metabolism and renal elimination (Li et al, 2015; Lin et al, 2009). Since *in vitro* studies suggested improved metabolic stability for retro-inverso peptides, their preliminary pharmacokinetic properties were tested *in vivo* by administrating compounds at a single dose level peripherally (subcutaneously and intravenously) and then the presence of compounds in plasma was determined 0.5 after peripheral dosing.

### Materials and Methods

Test compounds were subcutaneously (s.c.) or intravenously (i.v.) administered to male Spraque-Dawley rats (about six weeks old, n=3 per compound) at 0.5 mg/kg. Blood samples were taken via venipuncture (150 µl blood for 50 µl plasma) to EDTA tubes at 0.5 h after compound administration. Immediately following the sampling, the blood samples were centrifuged for plasma separation (within 30 min: 2700 G, 10 min). The samples were prepared by mixing 50 µL of plasma sample with 50 µL of internal standard solution (100 ng/ml of Enalapril for s.c or Phenacetine and Leu-Encephalin for i.v. in acdtonitrile with 1% of formic acid) and mixed. The samples were mixed for 3 minutes (1600 rpm), centrifuged for 20 minutes at 4000 rpm, supernatant, 50 µl, was diluted with 100 µl of 0.5% formic acid and submitted to LC-MS analysis. The standard samples were prepared into rat plasma by spiking the matrix into concentrations 0.1 - 10 000 ng/ml of the analyte, respectively, and otherwise treated as the samples. The analytical method was optimized for reaction monitoring chromatographic (peak shape & retention) shifts and mass spectrometric properties (ionization efficiency, MS/MS detection) without comprehensive prevalidation, but with tests on the detection limit, selectivity and matrix effects. The method performance (intra-assay accuracy & precision and range) was evaluated during the sample analysis using the back-calculated data from the standard samples (>6 concentrations).

Table 1 discloses *in vivo* pharmacokinetic properties of retro-inverso compounds after peripheral administration to rats. Compound concentration in plasma (Cp; ng/ml) at 0.5 h after intravenous (i.v.) and subcutaneous (s.c.) administration. Peripherally administrated parent compounds were not detected in plasma at the 0.5 h time point. Data are presented as mean +/- SEM (n=3).

Test compounds were subcutaneously (s.c.) and intravenously (i.v.) administered to male Spraque-Dawley rats (n=3) at 0.5 mg/kg. Administered compounds were compound 1 (SEQ ID NO: 1), compound 2 (SEQ ID NO: 2), compound 4 (SEQ ID NO: 4), compound (SEQ ID NO: 6) and compound 8 (SEQ ID NO: 8). Plasma samples were collected at 0.5 h post dosing followed by LC-MS/MS analysis. None of the tested parent compounds (SEQ ID NO:s 1, 5, 9) was detected in plasma at 0.5 h after administration indicating rapid elimination. Retro-inverso compounds demonstrated improved blood retention behavior after systemic peripheral administration, all tested retro-inverso compounds were detected at high ng/ml levels in plasma 0.5 h after administration.

**Table 1. In vivo pharmacokinetic of retro-inverso compounds after peripheral administration to rats. Data are presented as mean +/- SEM (n=3).**

| **Compound ID** | **Administration route** | **Modification** | **Cp at 0.5h (ng/ml)** | |
|---|---|---|---|---|
| | | | **Mean** | **SEM** |
| compound 1 | s.c. | parent | not detected | |
| compound 2 | s.c. | retro-inverso | 778.20 | 71.29 |
| compound 2 | i.v | retro-inverso | 359.33 | 101.11 |
| compound 4 | i.v | retro-inverso | 775.17 | 28.77 |
| compound 6 | s.c. | retro-inverso | 400.34 | 14.66 |
| compound 6 | i.v | retro-inverso | 465.79 | 12.35 |
| compound 8 | i.v | retro-inverso | 211.74 | 10.51 |

### CITATION LIST

### Patent literature

EP 58,481;
US 3,773,919;
WO 2007068803;
WO 2009133247;
WO 2013/3034805;
WO 2018/202957

### Non-patent literature

Airavaara M, Shen H, Kuo CC, Peranen J, Saarma M, Hoffer B, and Wang Y. 2009. Mesencephalic astrocyte-derived neurotrophic factor reduces ischemic brain injury and promotes behavioral recovery in rats. J. Comp. Neurol. 515(1):116-24.
Airavaara M, Harvey BK, Voutilainen MH, Shen H, Chou J, Lindholm P, Lindahl M, Tuominen RK, Saarma M, Hoffer B, Wang Y. 2012. CDNF protects the nigrostriatal dopamine system and promotes recovery after MPTP treatment in mice. Cell Transplant. 21(6):1213-23.
Bertolotti A, Zhang Y, Hendershot LM, Harding HP, Ron D. 2000 Dynamic interaction of BiP and ER stress transducers in the unfolded-protein response. Nat Cell Biol. 2(6):326-32.
Callizot N, Combes M, Steinschneider R, Poindron P. 2013. Operational dissection of beta-amyloid cytophathic effects on cultured neurons. J Neurosci Res. 91(5):706-16
Di L. 2014. Strategic approaches to optimizing peptide ADME properties. AAPS J. 17(1):134-43.
Dornburg R. 1995. Reticuloendotheliosis viruses and derived vectors. Gene Therap. 2: 301-310.
Fletcher JM and Hughes RA. 2006. Novel monocyclic and bicyclic loop mimetics of brain-derived neurotrophic factor. J. Pept. Sci. 12:515-524.
Glembotski CC. 2011. Functions for the cardiomyokine, MANF, in cardioprotection, hypertrophy and heart failure. J. Mol. Cell. Cardiol. 51:512-517.
Hefti F. Neurotrophic factor therapy for nervous system degenerative diseases. 1994. J Neurobiol., 25:1418-1435.
Hellman M, Arumae U, Yu LY, Lindholm P, Peranen J, Saarma M, and Permi P. 2011. Mesencephalic astrocyte-derived neurotrophic factor (MANF) has a unique mechanism to rescue apoptotic neurons. J.Biol.Chem. 286:2675-2680.
Huttunen, HJ and Saarma M. 2019. CDNF protein therapy in Parkinson's disease. Cell Transplantation 1-18.
Langer R, Brem H, Tapper D. 1981. Biocompatibility of polymeric delivery systems for macromolecules. J. Biomed. Mater. Res. 15: 167-277
Langer R. 1982. Controlled release of macromolecules. Chem. Tech., 12:98-105
Lindholm P., Voutilainen M.H., Lauren J., Peranen J., Leppanen V.M., Andressoo J.O., Lindahl M., Janhunen S., Kalkkinen N., Timmusk T., Tuominen R.K., and Saarma M. 2007. Novel neurotrophic factor CDNF protects and rescues midbrain dopamine neurons in vivo. Nature. 448:73-77.
Lindholm P, and Saarma M. 2010. Novel CDNF/MANF family of neurotrophic factors. Dev.Neurobiol. 70:360-371.
Lin JH. 2009. Pharmacokinetics of biotech drugs: peptides, proteins and monoclonal antibodies. Curr Drug Metab. 10(7):661-91.
Li Y, Wang Y, Wei Q, Zheng X, Tang L, Kong D, Gong M. 2015. Variant fatty acid-like molecules Conjugation, novel approaches for extending the stability of therapeutic peptides. Sci Rep. 5:18039.
Nadella R, Voutilainen MH, Saarma M, Gonzalez-Barrios JA, Leon-Chavez BA, Jiménez JM, Jiménez SH, Escobedo L, Martinez-Fong DJ. 2014. Transient transfection of human CDNF gene reduces the 6-hydroxydopamine-induced neuroinflammation in the rat substantia nigra. Neuroinflammation. 16;11:209.
Remington's Pharmaceutical Sciences, 22nd edition, Allen, Loyd V., Jr, Ed., (2012)
Sousa-Victor P, Jasper H, and Neves J. 2018. Trophic Factors in Inflammation and Regeneration: The Role of MANF and CDNF. Front. Physiol. 9:1629.
Tuschl T. 2002. Expanding small RNA interference. Nat. Biotechnol, 20: 446-448.
Visanji NP, Orsi A, Johnston TH, Howson PA, Dixon K, Callizot N, Brotchie JM and Rees DD. 2008. PYM50028, a novel, orally active, nonpeptide neurotrophic factor inducer, prevents and reverses neuronal damage induced by MPP+ in mesencephalic neurons and by MPTP in a mouse model of Parkinson's disease. FASEB J., 22(7):2488-97.
Voutilainen MH, Back S, Peranen J, Lindholm P, Raasmaja A, Mannisto PT, Saarma M, and Tuominen RK. 2011. Chronic infusion of CDNF prevents 6-OHDA-induced deficits in a rat model of Parkinson's disease. Exp. Neurol. 228:99-108.
Voutilainen MH, Back S, Porsti E, Toppinen L, Lindgren L, Lindholm P, Peranen J, Saarma M, and Tuominen RK. 2009. Mesencephalic astrocyte-derived neurotrophic factor is neurorestorative in rat model of Parkinson's disease. J.Neurosci. 29:9651-9659.
Wang M, Kaufman RJ. 2016. Protein misfolding in the endoplasmic reticulum as a conduit to human disease. Nature 529(7586):326-35.
Xue Q, Liu Y, Qi H, Ma Q, Xu L, Chen W, Chen G, Xu X. 2013. A novel brain neurovascular unit model with neurons, astrocytes and microvascular endothelial cells of rat. Int J Biol Sci. 9(2):174-89.
Yan Y, Rato C, Rohland L, Preissler S, Ron D. 2019. MANF antagonizes nucleotide exchange by the endoplasmic reticulum chaperone BiP. Nat Commun. 10(1):541.
Zhao H, Liu Y, Cheng L, Liu B, Zhang W, Guo YJ, Nie L. 2013. Mesencephalic astrocyte-derived neurotrophic factor inhibits oxygen-glucose deprivation-induced cell damage and inflammation by suppressing endoplasmic reticulum stress in rat primary astrocytes. J Mol Neurosci. 51(3):671-8

## Claims

1. A peptide having a length of 8 - 32 amino acids or a pharmaceutically acceptable salt thereof comprising a retro-inverso form of an amino acid sequence of C-X₁-X₂-X₃-C (SEQ ID NO: 17),
wherein
X₁ is selected from the group consisting of R, K, I, G, A and S;
X₂ is absent or selected from the group consisting of G, A, R, K, I and S; and
X₃ is selected from the group consisting of A, G and S.

2. The peptide according to claim 1, comprising a retro-inverso form of an amino acid sequence of E-X₄-C-X₁-X₂-X₃-C-A-E (SEQ ID NO: 18),
wherein
X₁ is selected from the group consisting of R, K, I, G, A and S;
X₂ is absent or selected from the group consisting of G, A, R, K, I and S;
X₃ is selected from the group consisting of A, G and S; and
X₄ is selected from the group consisting of E, T, V, D, M and G.

3. The peptide according to claim 1 or 2, comprising a retro-inverso form of an amino sequence of X₅-X₆-X₇-X₈-E-X₄-C-X₁-X₂-X₃-C-A-E-X₉-X₁₀-X₁₁ (SEQ ID NO: 19),
wherein
X₁ is selected from the group consisting of R, K, I, G, A and S;
X₂ is absent or selected from the group consisting of G, A, R, K, I and S;
X₃ is selected from the group consisting of A, G and S;
X₄ is selected from the group consisting of E, T, V, D, M and G;
X₅ is absent or selected from the group consisting of H, D, Q, R, Y, N and S;
X₆ is absent or selected from the group consisting of S, D, G, N and R;
X₇ is absent or W;
X₈ is absent or G;
X₉ is absent or K;
X₁₀ is absent or selected from the group consisting of T, S, A, I and N; and
X₁₁ is absent or selected from D and E.

4. The peptide according to any one of claims 1 - 3, comprising a retro-inverso form of an amino acid sequence of X₁₂-X₁₃-X₁₄-X₁₅-X₁₆-X₁₇-X₁₈-X₁₉-X₂₀-X₂₁-X₂₂-X₂₃-V-X₂₄-E-L-K-X₂₅-X₂₆-L-X₅X₆-X₇-X₈-E-X₄-C-X₁-X₂-X₃-C-A-E-X₉-X₁₀-X₁₁ (SEQ ID NO: 20),
wherein
X₁ is selected from the group consisting of R, K, I, G, A and S;
X₂ is absent or selected from the group consisting of G, A, R, K, I and S;
X₃ is selected from the group consisting of A, G and S;
X₄ is selected from the group consisting of E, T, V, D, M and G;
X₅ is absent or selected from the group consisting of H, D, Q, R, Y, N and S;
X₆ is absent or selected from the group consisting of S, D, G, N and R;
X₇ is absent or W;
X₈ is absent or G;
X₉ is absent or K;
X₁₀ is absent or selected from the group consisting of T, S, A, I and N; and
X₁₁ is absent or selected from D and E,
X₁₂ is absent or selected from the group consisting of L, I and V;
X₁₃ is absent or D;
X₁₄ is absent or selected from L and W;
X₁₅ is absent or selected from the group consisting of A, S, T, E and N;
X₁₆ is absent or selected from S and T;
X₁₇ is absent or selected from V and D;
X₁₈ is absent or selected from D and A;
X₁₉ is absent or L;
X₂₀ is absent or selected from the group consisting of R, K, S and W;
X₂₁ is absent or K;
X₂₂ is absent or selected from the group consisting of M, L, I and V;
X₂₃ is absent or R;
X₂₄ is selected from the group consisting of A, K, T, L and V;
X₂₅ is selected from the group consisting of Q, K and R; and
X₂₆ is selected from I and V.

5. The peptide according to any one of claims 1 - 4, consisting of a sequence selected from the group consisting of:
KEACARCEEGWSHLIQKLEAVRM (SEQ ID NO: 2),
KEACGKCTEGWDDLIKKLEKVRL (SEQ ID NO: 4),
TKEACARCEEG (SEQ ID NO: 6),
SKEACGKCTEG (SEQ ID NO: 8),
TKEACAGRCEEG (SEQ ID NO: 10),
SKEACGGKCTEG (SEQ ID NO: 12),
EACARCEE (SEQ ID NO: 14), and
EACGKCTE (SEQ ID NO: 16),
wherein all amino acids of the peptide are D-amino acids.

6. The peptide according to any one of claims 1 - 5, wherein the peptide binds to GRP78.

7. The peptide according any one of claims 1 - 6, wherein cysteine is in a reduced form or in disulphide bridged form.

8. The peptide according to any one of claims 1 - 7, wherein the N-terminus of the peptide is acetylated.

9. The peptide according to any one of claims 1 - 8, wherein the C-terminus of the peptide is amidated.

10. The peptide according to any one of claims 1 - 9, wherein the N-terminus of the peptide is acetylated and the C-terminus of the peptide is amidated.

11. The peptide according to any one of claims 1 - 10, wherein the peptide is a pseudopeptide.

12. The peptide according to any one of claims 1 - 11, wherein the peptide is a cyclic peptide.

13. The peptide according to any one of claim 1 - 12 conjugated to a detectable chemical moiety, a biochemical moiety, or polyethylene glycol (PEG).

14. The peptide of any one of claims 1 - 13, wherein the peptide has at least one of the following properties:
(i) can dose-dependently protect TH-positive neurons from MPP+ toxicity;
(ii) reduces the number of a-synuclein inclusions in TH-positive neurons;
(iii) has improved stability in plasma compared to its parent counterpart;
(iv) has improved stability in hepatocytes compared to its parent counterpart; or
(v) has improved ability to pass through the blood brain barrier compared to its parent counterpart.

15. The peptide according to any one of claims 1 - 14 for use as a medicament.

16. The peptide according to any one of claims 1 - 14 for use in the treatment of a degenerative disease or disorder, a chronic disease or disorder, or a progressive disease or disorder, such as a neurodegenerative disease or disorder.

17. The peptide for use according to claim 16, wherein said neurodegenerative disease or disorder is a central nervous system disease selected from the group consisting of Parkinson's disease, Alzheimer's disease, multiple system atrophy, amyotrophic lateral sclerosis, frontotemporal lobar degeneration, dementia with Lewy bodies, mild cognitive impairment, Huntington's disease, traumatic brain injury, traumatic spinal cord injury, progressive supranuclear palsy, Pick's disease, pure autonomic failure, corticobasal degeneration, chronic traumatic encephalopathy, spinocerebellar ataxia, and peripheral neuropathy.

18. The peptide according to any one of claims 1 - 14 for use in the treatment of a monogenic hereditary disease having ER stress as a pathogenic component selected from the group consisting of Wolcott-Rallison syndrome, Wolfram syndrome, Marinesco-Sjögren syndrome, Machado-Joseph disease, and degenerative retinal diseases such as retinitis pigmentosa, and inherited nephrotic syndromes such as primary nephrotic syndrome and autosomal dominant polycystic kidney disease.

19. The peptide for use according to any one of claims 15 - 18, wherein said peptide is administered by peripheral administration such as intravenous, intraarterial, subcutaneous, intranasal, intraocular, intratympanic, or topical administration, enteral, parenteral or topical routes including oral, rectal, sublingual or buccal administration, intraperitoneal, intramuscular, intraarticular, transdermal, intracochlear, topic ocular, or inhalational administration, or intracranial, intrathecal, epidural or intralesional administration.

20. A pharmaceutical composition comprising the peptide according to any one of claims 1 - 14 and at least one of the following: a pharmaceutically acceptable carrier, a pharmaceutically acceptable excipient, a preservative, a stabilizer and/or a diluent.

21. The pharmaceutical composition according to claim 20 for use as a medicament.

22. A pharmaceutical composition according to claim 20, for use in the treatment of a degenerative, chronic or progressive disease or disorder, such as a neurodegenerative disease or disorder.

23. The pharmaceutical composition for use according to claim 22, wherein said neurodegenerative disease or disorder is a central nervous system disease selected from the group consisting of Parkinson's disease, Alzheimer's disease, multiple system atrophy, amyotrophic lateral sclerosis, frontotemporal lobar degeneration, dementia with Lewy bodies, mild cognitive impairment, Huntington's disease, traumatic brain injury, traumatic spinal cord injury, progressive supranuclear palsy, Pick's disease, pure autonomic failure, corticobasal degeneration, chronic traumatic encephalopathy, spinocerebellar ataxia, and peripheral neuropathy.

24. The pharmaceutical composition according to claim 20 for use in the treatment of a monogenic hereditary disease having ER stress as a pathogenic compound selected from the group consisting of Wolcott-Rallison syndrome, Wolfram syndrome, Marinesco-Sjögren syndrome, Machado-Joseph disease, and degenerative retinal diseases such as retinitis pigmentosa, and inherited nephrotic syndromes such as primary nephrotic syndrome and autosomal dominant polycystic kidney disease.

25. The pharmaceutical composition for use according to any one of claims 20 - 24, wherein said composition is administered by peripheral administration such as intravenous, intraarterial, subcutaneous, intranasal, intraocular, intratympanic, or topical administration, enteral, parenteral or topical routes including oral, rectal, sublingual or buccal administration, intraperitoneal, intramuscular, intraarticular, transdermal, intracochlear, topic ocular, or inhalational administration, or intracranial, intrathecal, epidural or intralesional administration.
